(19) Europäisches Patentamt

European Patent Office

Office européen des brevets

(11) Publication number: 0 558 566 B1

(12) EUROPEAN PATENT SPECIFICATION

(45) Date of publication of patent specification: 14.12.94

(51) Int. Cl.5: **C12Q 1/00**, G01N 33/48, G01N 33/84

(21) Application number: 91920230.9

(22) Date of filing: 15.10.91

(86) International application number:
PCT/SE91/00693

(87) International publication number:
WO 92/08803 (29.05.92 92/12)

(54) A METHOD FOR IN VITRO ANALYSIS OF METAL-INDUCED ALLERGIES.

(30) Priority: 20.11.90 SE 9003695

(43) Date of publication of application:
08.09.93 Bulletin 93/36

(45) Publication of the grant of the patent:
14.12.94 Bulletin 94/50

(84) Designated Contracting States:
AT BE CH DE DK ES FR GB GR IT LI NL SE

(56) References cited:
EP-A- 90 359
EP-A- 99 812
EP-A- 140 379
EP-A- 265 411
GB-A- 2 230 790

DERWENTS ABSTRACTS, no. 89-376
630/51&NUM;

DERMATOLOGICA, vol. 171, 1985; R. SCHUP-
PLI, pp. 158-162&NUM;

(73) Proprietor: STEJSKAL, Vera
Axel Johnsons väg 14
S-182 31 Danderyd (SE)

(72) Inventor: STEJSKAL, Vera
Axel Johnsons väg 14
S-182 31 Danderyd (SE)

(74) Representative: Fagerlin, Heléne et al
H. ALBIHNS PATENTBYRA AB
P.O. Box 3137
S-103 62 Stockholm (SE)

**Description**

The present invention describes a method for in vitro analysis of metal-induced allergies, more specifically of antigen-specific lymphocytes, so called memory cells. The procedure is called MELISA (Memory Lymphocyte Immuno-stimulation Assay). Analysis made with the help of MELISA has shown a great degree of clinical relevance.

There exists a great demand for the technique which could diagnose the agents, so called antigens which cause allergies. This applies for example to subjects who work in chemical industry and who come in contact with substances, such as drugs (for example antibiotics) or metals, such as nickel and mercury. It is generally accepted that the number of allergic subjects has increased during the recent years. If the proper allergen is determined in an individual sensitive to this particular allergen, the subject can avoid the contact and thus diminish the risks for the recurrence of symptoms.

A known method for the detection of the allergens has been a so called patch test. In this test various test compounds are applied on the skin of the back of the subject supposed to be allergic to tested compounds. 48 - 72 hours later the occluded region of the skin is examined and evaluated by experienced dermatologist. The reaction is considered to be positive if an inflammation is seen at the place of a patch application. This method has, however, several disadvantages. For example, mercury compounds are corrosive (toxic) for the skin and false-positive irritative reactions are quite common. Further on, irritative reactions are difficult to distinguish from immunologically specific allergic reactions. The prevalency of mercury allergy based on the positive patch test varies widely, from 6% to 62%. (Finne K., Göransson K.,Winckler L.,Int. J. 0ral Surg.,11:236-9,1982; Stenman E.,Bergman M, Scand. J. Dental Res.,97:76-82, 1989; Lundström IMC,Int. J. 0ral Surg., 13:16-24, 1984; Fisher AA,Cutis, 110, 112, 117,1985.)

Another disadvantage of the patch test is that each application of reactive chemicals on the skin may increase the immune reactivity of the individual to a given antigen (boosting effect) and thus aggravate the allergic symptoms. Thus, the patch testing with mercury, cobalt, formaldehyde and epoxides cannot be recommended for all patients. The Food and Drug Administration (FDA) has recently received a report on side effects of patch tests, performed to diagnose hypersensitivity against mercury (Food and Drug Administration, National Centre for Devices and Radiological Health, Device Monitoring Branch, Device Experience Network Monthly Report, 08:11-12,1983.) Another method, used previously, is the so called LTT method, Lymphocyte Transformation Test, sometimes also called Lymphocyte Stimulation Test (LST). Transformation means that small lymphocyte grows and thus transforms to a large lymphoblast. This process may be initiated by certain surface-active substances which non-specifically stimulate lymphocytes from all individuals to division (mitosis). The same reaction can be observed in the blood of animals or individuals exposed in vivo to a particular antigen if the same antigen is added in vitro. LTT measures the immune response of the individual against a given antigen, for example against virus or bacteria.

LTT has previously been used more or less succesfully for the diagnostics of certain types of hypersensitivity reactions, so called allergies. It has mostly been used for delayed-type of hypersensitivity reactions, also called type 4 reactions. These reactions are known to be cell-mediated and participate for example in contact dermatitis (eczema). The antigens most frequently studied have been penicillins and heavy metals such as nickel but also cobalt, gold, beryllium and inorganic mercury. Both mitogenic and antigenic properties of these substances have been found with this technique. As far as mercury is concerned the LTT technique has not been able to dissociate among those two properties.

In the conventional LTT, heparinized blood is separated on density gradient and monocyte-rich lymphocyte suspension is isolated. Granulocytes and red blood cells sediment at the bottom of the centrifugation tube. Lymphocyte suspension is cultivated with the antigens aimed to be studied in microplates (96 wells) in 0,2 ml media containing 200,000 cells for 4-5 days. After cultivation, the presence of lymphoblasts is measured by addition of radio-labeled thymidine or of other aminoacid incorporated into nucleic acids during cell division in vitro.

It has generally been anticipated that inorganic mercury compounds such as mercury chloride ($HgCl_2$) function as a mitogen for human and animal lymphocytes in culture (Schöpf E.,Schulz KH,Gromm, Naturwissenschaften, 21:568-9, 1967). Thus, in these studies, lymphocytes from mercury patch test-positive subjects but also from mercury patch test-negative subjects were stimulated. Patch test positivity was taken as an evidence for the presence of mercury allergy. Since lymphocytes from both allergic and non-allergic people were stimulated, no further attempts were performed to examine the possibility to use mercury-specific lymphocytes as an indicators of mercury allergy. Interestingly, no studies were ever published using lymphocytes from individuals lacking amalgam fillings (amalgam is an alloy containing mercury). Schopf and coworkers (Die Naturwissenschaften,p. 461-465, 1969, Schopf E., Schulz KH, Insee I.Arch. Klin.Exp. Derm, 234:420-433,1969) have reported that many organomercurials such as phenyl mercury, ethyl mercury and methoxyethyl mercury did not stimulate lymphocytes in vitro. Other organic mercurials such as dibrom-hydroxy mercury were stimulatory. These inconsistent data and previously mentioned "mitogenicity" of mercury chloride resulted in the cease of the attempts to use LTT for diagnosis of mercury allergy in vitro.

During the work on the present invention it has become evident that the LTT method may give false-positive or false-negative $^3$H-thymidine incorporation due to macrophages and monocytes present in lymphocyte fraction. The radioactivity measured does not necessarily reflect the real frequency of antigen-specific lymphocytes - so called memory cells. By an autoradiography it has been shown that macrophages from some individuals incorporate the isotope in the nucleus and thus, macrophage DNA is extracted together or instead of lymphoblast DNA on filtration papers. Last but not least, macrophages can also phagocyte and store in the cytoplasma isotope-containing cell debris. Since this is a rapid process, this may result in depletion of isotope available for lymphoblast labelling. Further on, stimulated macrophages contain certain enzymes which degrade radioactively labeled thymidine, thus preventing the radioactive measurement of stimulated lymphocytes. Despite of the fact that lymphoblasts may be present in such cultures, they cannot be shown by radiolabelling only.

The present invention, MELISA, describes a method for detection of metal allergies in vitro with the help of lymphocytes, so called memory cells, in the blood in which lymphocytes are cultivated in the presence of antigen with the aim of detection of memory cells for the specific antigen among the other lymphocytes, since antigen-specific lymphocytes are stimulated to division which is measured. The procedure is characterized by the depletion of monocytes and macrophages from lymphocyte fraction and by the cultivation of lymphocytes with one or several antigens.

The antigens may be any substance that is not endogenic i.e. not naturally present in the human body such as heavy metals, in the form of organic or inorganic metal compounds. These metals may be mercury, tin, titan, copper, cobalt, chrome, nickel, cadmium, antimony and barium, aluminium, tin, lead, gold, silver, lead, arsenic, palladium, platinum. Compounds used in dental fillings or prothesis in the human body may also be tested according to the invention. They may be ceramics, glass ionomers, pigments, constituents of composite materials of and in root filling material such as gutta percha composite materials. The invention is especially used for testing mercury allergy with organomercurials and with low concentrations of inorganic mercurials. (For $HgCl_2$, the low concentration is less that 0,5 micrograms per ml media).

In addition to the radioactive technique which is used for an objective measurement of memory cell division, a direct microscopic evaluation of lymphoblasts in cultures is performed. Since the new procedure according to invention, unlike Lymphocyte Transformation Test, reliably measures antigen-specific lymphoblasts, derived from memory cells, the technique is called MELISA (Memory Lymphocyte Immuno-Stimulation Assay). During the MELISA development and during the studies of memory cells formed against low molecular substances we have not found any evidence of non specific mitogenic effects caused by substances studied by us if experiments were run under proper conditions.

The procedure starts from venous blood which is prevented from coagulation. This can be achieved by the addition of anticoagulants to the blood. However, it has been shown that heparin, EDTA, and a majority of citrates interferes with the analysis, probably by suppression of hapten presentation. In some experiments, ADC solution (Becton Dickinson Vacutainer Systems, Rutherford, New Jersey 070 70) worked succesfully. Preferably, the blood is defibrinated by hand shaking or mechanically together with the particles. Preferably, the blood is shaken approx. 5 - 10 minutes together with glass or plastic beads. Particularly, the blood is collected in 10 ml vacutainer sterile tubes with plastic beads (Becton Dickinson) and shaken for about 6 minutes.

Lymphocyte fraction is isolated thereafter through gradient centrifugation. This can be achieved by layering defibrinated blood on Leuco PREP[(R)] (Becton Dickinson) and centrifugation 1300-1600 g for 10-20 minutes, preferably at 1500 g for approx. 15 minutes. One can also dilute the blood at least twice, preferably with isotonic solution, especially with a culture medium such as RPMI 1640-medium (Gibco, UK, Roswell Park Memorial Institute, containing 10 mmol of N'-2-hydroxyethylpiperazine-N'-2-ethansulfonic acid and 1 gram of $NaHCO_3$ per liter). To the culture medium, the antibiotics such as gentamycine and also glutamine is preferably added. The diluted blood is applied over a density gradient, preferably over Ficoll-Paque (Ficoll-Paque contains per 100 ml Ficoll 400 5.7 g natrium-diatrizoate, 9 g natrium calcium edetate, Pharmacia) in the sterile centrifugation tubes. The mixture is centrifuged for 20-45 minutes at 400-800 g, preferably for 30 minutes at 600 g. The proper relative ratio between RPMI-diluted blood and Ficoll is 6:4.

After centrifugation, the lymphocyte fraction containing monocytes and other antigen-presenting cells is isolated and washed eventually with isotonic solution or with medium such as RPMI 1640-medium.

Monocytes and other adherent cells are then removed from the lymphocyte fraction by treatment with substances which are toxic for monocytes, for example with lysosome-tropic agents (L-leucine methyl ester,

3

or structurally related substances). The development of monocytes into macrophages can also be prevented by the addition of adenosine or structurally related compounds. Preferably, monocytes are removed by adherence to plastics, glass or other tissue compatible sterile surfaces. Lymphocyte fraction is suspended in RPMI 1640 medium and is incubated on sterile tissue compatible surfaces for 10 minutes overnight, preferably as long as antigen-presenting cells adhere to the surface. This step can be monitored under the invert microscope. Preferably, the adherence is performed for 1 hour at plastic bottle at 36-38°C. During this period, the majority of monocytes and other antigen-presenting non lymphocytic cells will adhere to the surface and can easily be removed from lymphocyte suspensions. As a cultivation medium RPMI 1640 medium containing heat-inactivated (56° C, 30 minutes) human AB RH+ serum or plasma, preferably 20% inactivated AB+ serum is used. Non adherent lymphocytes are collected and diluted in cultivation medium. Cells are counted preferably by mixing with Gentiana violett according to Turk KLVI (Apoteksbolaget, Malmö) and the number of cells is counted in the microscope in a Burker chamber (Kebo lab, Stockholm, or in an automatic cell counter). The cells are diluted to 0,5-2x10$^6$ cells per ml, preferably 1x10$^6$ cells per ml. A cultivation medium, preferably RPMI 1640 medium containing 7-15% of heat-inactivated human AB+ serum, (preferably 10% AB+ serum) is used for dilution.

Lymphocytes are delivered into sterile round bottomed culture tubes, preferably 10 ml tubes together with the antigen solution to be tested. Sterile 48-wells plates can be also used for cultivation. Test substances (the antigens) can be freshly delivered or lyophilized directly on the plates. Such plates can be stored with convenience. The final cell concentration is 0,5-3 x10$^6$ cells, preferably 1x10$^6$ cells per culture. The cultures are incubated for 4-8 days, preferably for 5 days. After cultivation, lymphocyte transformation is analysed by radioactive isotope incorporation into lymphoblast DNA and/or by morphological evaluation under the microscope. The lymphocyte proliferation may also be evaluated with flow cytometry. Monoclonal antibodies against human T- and B-lymphocytes combined with monoclonals against proliferative cells may be used.

Evaluation of DNA-synthesis after cultivation in tubes

For evaluation of DNA incorporation, microplates with 96 wells are used. To each well, 30-60 kBq of labeled thymidine or other functionally similar aminoacid is added, preferably 37,0 kBq methyl 3H-thymidine (specific activity 3.18 TBq mmol). 100-250 microliters of cell suspension in triplicate is transferred from each culture, preferably 200 microliters. Plates are incubated at 36-38°C, preferably at 37°C for 3-6 hours in C0$_2$-incubator. After incubation the radioisotope-containing DNA is harvested. Preferably, a fully automatic cell harvester such as INOTECH AG, Wohlen, Schweiz, is used. Distilled water is used for washing procedure. The plates can be harvested directly or they can be stored overnight or several days before the harvest. Plates can even be frozen for considerable length of time.

Evaluation of DNA synthesis after cultivation in 48-well plates

75-150 kBq methyl-3H-thymidine (specific activity 3,18 TBq/mmol) or another functionally similar aminoacid,is delivered into each of 48 wells (preferably 111 kBq per well). The cellular sediment in cultivation plates is resuspended and 300-800 microliters, preferably 600 microliters from each culture is removed and transferred to the labeled 48-well plate. Incubation and harvesting proceeds as described above.

After harvest, filter discs containing lysed cells are removed and placed into microampulles. 1ml of Opti-Scint (LKB) is added. The ampoules are closed and the radioactivity is then measured in the scintillation counter. One can also measure DNA directly of the dry filters without microampoules and scintilation fluid in for example Tracer 96 Counting unit (INOTECH AG).

For morphological evaluation under the microscope, 50-100 microliters of cultivated cells, preferably 75 microliters, is centrifuged in a cytocentrifuge at 70-100 g under the 1-10 minutes, preferably at 90 g for 2 minutes. Cell preparations are fixed preferably with methanol or other functionally similar fixatives and are stained with May-Grunwald och Giemsa (Diagnostica, Merck). A semiquantitative evaluation of the number of lymphoblasts (transformed lymphocytes) and of macrophages is performed under the microscope. During the microscopic analysis, general condition of cultivated cells is observed and also number of macrophages and their stimulation grade is noted, especially if it differs from normal picture. In majority of experiments, there is a good correlation between DNA synthesis and number of stimulated lymphoblasts observed on cell smears.

DNA synthesis is expressed as number of counts per minute (CPM) for each culture. Stimulation of DNA synthesis by antigens in culture compared to DNA synthesis in control cultures is expressed as Stimulation Index:

Stimulation Index (SI) = CPM in antigen-treated culture divided by mean value of CPM in control cultures.

Increase in DNA synthesis can also be expressed as delta ($\Delta$) CPM, which has the following definition:

($\Delta$) CPM = CPM in antigen-treated cultures minus mean CPM in control cultures.

DNA synthesis in control cultures (cells were cultivated in the absence of antigen in medium only) can vary widely due to so called spontaneous activation of lymphocytes. CPM values less than 100 per 200 000 cells indicate suboptimal culture conditions. This can also be confirmed through morphological examination. High values (for example patient L16, Table 14A) indicate spontaneous blastogenesis but this also has to be confirmed by microscopy.

As a positive control of lymphocyte transformation at the time of each analysis (experiment), one can use 0.01-20 micrograms of PPD (Purified Protein Derivative, Tuberculin, Statens Serum Institute, Copenhagen, Denmark). Preferably, concentration 0.1 or 1 microgram per ml should be used. Other antigens of bacterial or viral origin to which human population mounts immune responses can also be used, for example Streptokinase-Streptodormase (SK-SD) or tetanus toxoid. The degree of stimulation induced by PPD varies among different individuals. In most experiments, however, PPD induces strong increase of DNA synthesis. Very rarely, PPD response is not observed and this can depend on genetical factors or on the patient's general health deterioration (for example due to cancer, AIDS and other immunosuppressive diseases). If PPD response is low or absent, it is important to find out if the patient has not been immunized against tuberculosis or if he is a genetic non responder. One way is to find out if patch test with Tuberculin was positive. The absent stimulation with PPD can also indicate treatment of the patient with immunosuppressants, such as corticosteroids or cytostatic drugs.

Low blastogenesis (lymphocyte proliferation in control cultures and thus low control values) can result in false-positive DNA incorporation (no lymphoblasts in cultures). On the other hand, strong spontaneous blastogenesis (lymphocyte transformation) results in low sensitivity of the test. The patient should be retested if the results are of doubtful significance.

The evaluation of the results in regard to stimulation of DNA is based on the following new criteria:

1. Stimulation which is at least twice as high as control value (background) is considered as weakly positive if obtained with concentrations which are known to be optimal for the tested substance. If there are several subsequent concentrations of the test substance which induce weak proliferation, the results are considered positive.

2. Stimulation which is at least 3 or more as the background is considered as positive.

3. Positive responses should preferably show dose-response relationship.

A semiquantitative analysis of the lymphoblasts is performed with the help of morphological evaluation under the microscope. The number of lymphoblasts detected in cell sample is given, if the number does not exceed 100. If more than 100 lymphoblasts are present, the actual data are not given and the observation is described as >100. Other observations, which can be important for the evaluation of the results, such as spontaneous blastogenesis, presence of large number of macrophages in cultures or suboptimal conditions in the culture (damaged or lysed lymphocytes) should also be noted.

Morphological information increases the knowledge about the general condition of cultured cells. Activated macrophages can sometimes mimic false-positive response because of incorporation of radiolabeled DNA-precursor. Cultures which contain only activated macrophages but not lymphoblasts should be considered negative despite of positive isotope incorporation into DNA.

Activated macrophages can through the phagocytosis contribute to false-negative results (discussed previously).

Since the new MELISA technique repeatedly removes monocytes and macrophages, the risk for false-positive and false-negative results decreases considerably.

According to the present invention most of the monocytes and other adherent cells are removed already at the beginning of the culture, since lymphocyte fraction is incubated on adhesive surfaces in the absence of test substance (1st adherence step).

MELISA is also preferably performed by cultivation of lymphocytes together with the test substance, so called antigen in the macroplates (1 ml-wells) or in tubes and thus this step is separated from the cultivation of lymphoblasts in the presence of radioactive DNA-precursor at the end of the culture. Thus, lymphocyte suspension is transferred from the cultivation plate or tube to the radioactive plate (2nd adherence step). During the cultivation period (4-5 days), the considerable amount of macrophages develops in the cultures and can be seen in cell smears despite of adherence performed at the start of culture. This means that a certain number of monocytes were non adherent at the time of first adherence step and could develop into macrophages later. According to LTT (LST) method, the cultivation with antigen and incorporation of radioactive precursor into DNA is invariably performed in the same plate (96-well plate). In most cases, no adherence step is performed at the beginning of culture before lymphocytes are cultivated in the presence of test substance. According to actual invention, monocytes, macrophages and other adherent cells are removed from the lymphocyte fraction by at least two consecutive adherence steps before radioactive DNA-precursor incorporation is performed.

In between the two consecutive adherence steps or between the last two of several adherence steps, if more than two adherence steps are performed, lymphocytes should be cultivated at least as long that remaining non adherent monocytes develop into the macrophages. This development can be observed under the invert microscope or on stained cell smears. Cultivation for 3-5 days is suitable.

The new invention is illustrated with the help of enclosed drawings where

Fig. 1 shows lymphocyte proliferation to inorganic and organic mercury compounds in patients with oral lichen.

Fig. 2 shows maximal stimulation indexes to inorganic and organic mercury in subjects tolerating amalgam without any side effects.

Fig. 3 shows lymphocyte proliferation to inorganic and organic mercury compounds in amalgam-free donors.

Fig. 4 shows lymphocyte proliferation to inorganic and organic mercury compounds in one patient with oral lichen. Lymphocyte proliferation is dose-dependent.

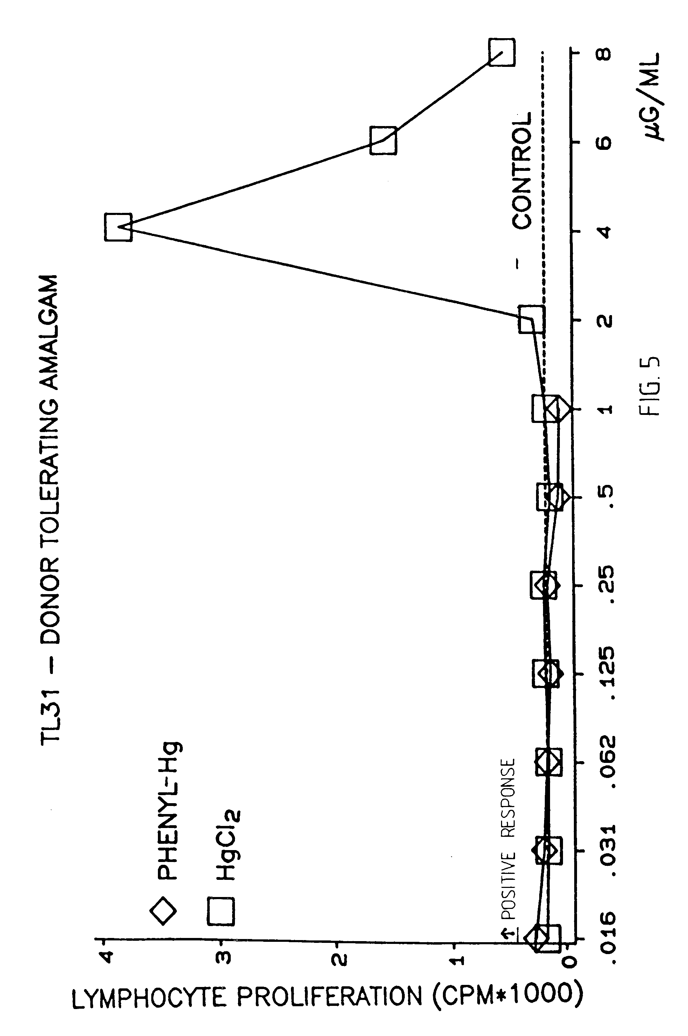

Fig. 5 shows lymphocyte proliferation to inorganic and organic mercury compounds in one donor tolerating amalgam.

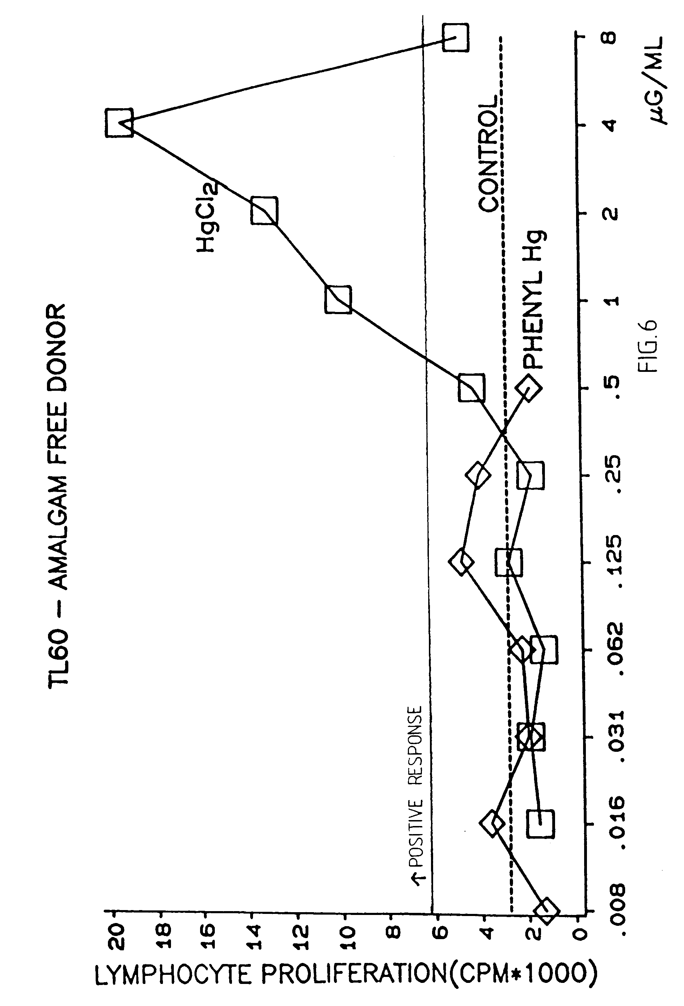

Fig. 6 shows lymphocyte proliferation to inorganic and organic mercury compounds in amalgam-free donor. Dose response curve to high concentrations of $HgCl_2$ is shown.

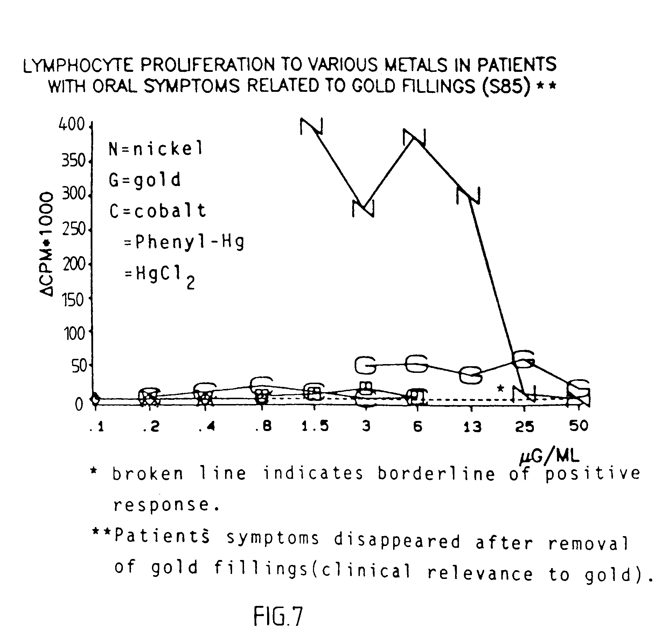

Fig. 7 shows lymphocyte proliferation to various metals in patients with oral symptoms related to gold fillings.

## Diagnosis of Mercury Allergy

### Patient and Controls

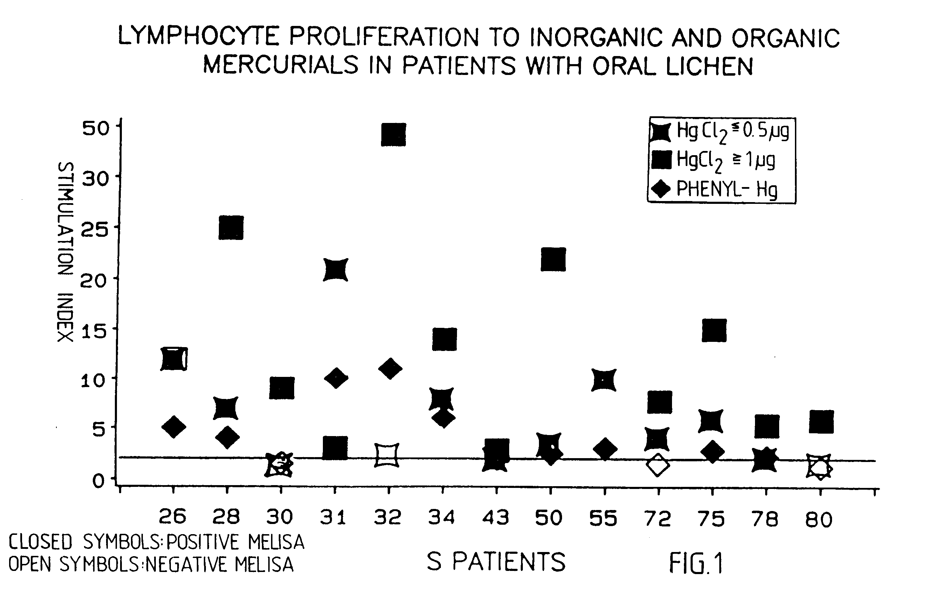

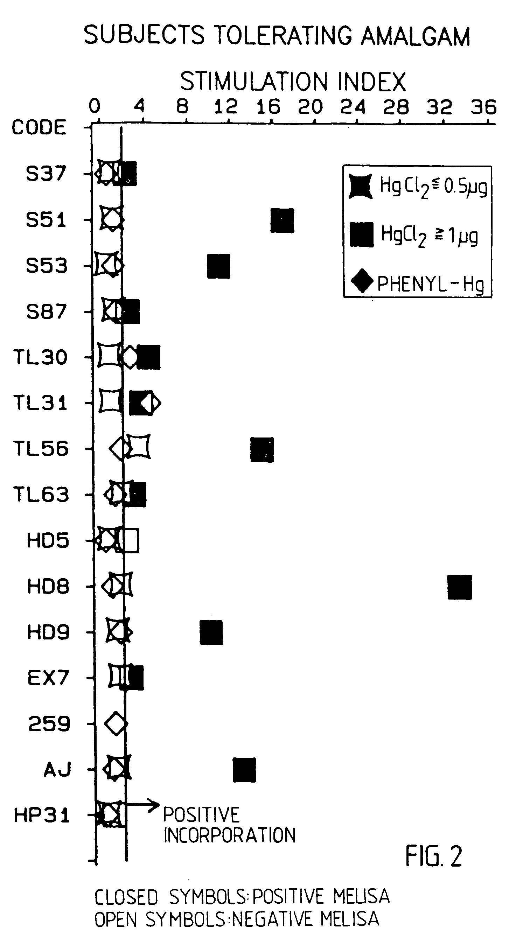

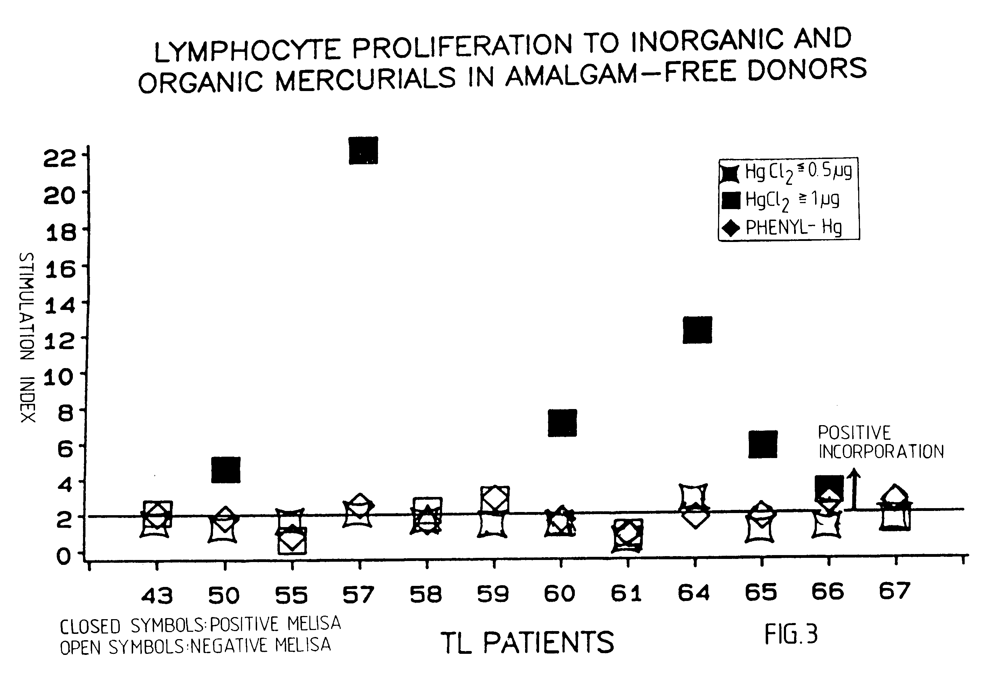

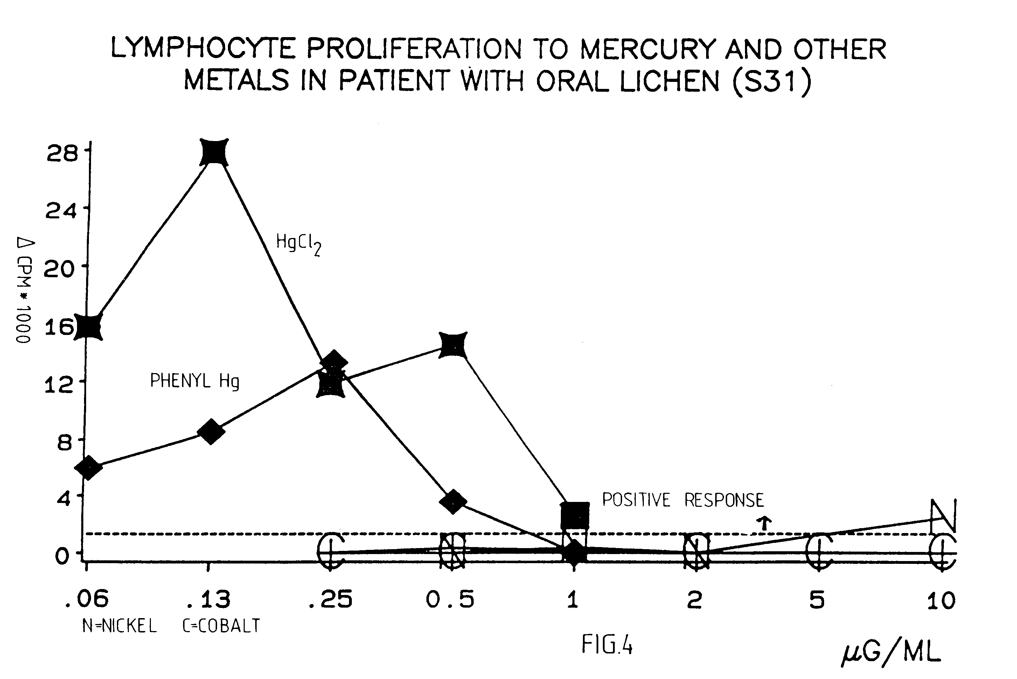

Patients suffering from lichenoid changes in oral mucosa adjacent to amalgam fillings (group I) and other patients who suspected mercury allergy as a contributive factor of their illnesses (L16,S31, S92 and other patients in Table 1 and Fig. 1 and 4) were examined with patch test and in in vitro MELISA test performed according to this new invention. MELISA was performed according to example 1 and 2. As test substance, phenylmercuric acetate, $HgCl_2$,methylmercuric chloride and ethylmercuric chloride were used. Healthy amalgam bearers (Fig.2 and for example donor SK45) and amalgam-free persons (TL patients,Fig.3) were similary examined.

Specific memory cells against phenylmercuric acetate were demonstrated in majority of patients suffering from lichenoid changes localized closely to amalgam fillings (10 out of 13 patients in Fig. 1). 3 patients lacked organic Hg-specific memory cells. Thus, their problems may have been caused by irritative-toxic effect of amalgam rather than by allergy to mercury. There may be also other reasons, such as allergy to other metals which are the part of the amalgam alloy. The cellular reactivity to organomercurials studied in these experiments was not found in healthy amalgam bearers and in amalgam-free donors, (Fig. 2 and Fig. 3). Thus, the presence of organomercurial-specific-memory cells correlates with the allergic symptoms (mucosal changes) and has clinical relevance to amalgam fillings.

All patients with oral mucosal problems studied in these experiments demonstrated memory cells to high concentrations of $HgCl_2$ (0,5 micrograms per ml or higher concentrations. Since 12 out of 15 symptom-free amalgam bearers and 7 out of 12 amalgam-free donors reacted in the same way, it has to be concluded that the presence of memory cells induced by high concentrations of $HgCl_2$ reflects immune response to mercury but not allergy. Positive patch test to phenylmercuric acetate and to Thimerosal (ethylmercuric thiosalicylate) has been demonstrated in 6 out of 13 patients with oral lichen. Many of these patients reacted also to Thimerosal in MELISA. Both tests showed clinical relevance to amalgam fillings. Thus, the patients' symptoms disappeared or diminished significantly following amalgam removal. Since memory lymphocytes circulate through the blood and lymphoid system of the whole body, they may with advantage be used for diagnostics of systemic mercury allergy.

Since lymphocyte reactivity to organomercurials was an eminent feature of memory cells from patients with pathological changes suspected to be caused by mercury and was not detected in healthy amalgam-exposed subjects and in amalgam-free persons, phenyl-mercury salts and other structurally related organomercurials can be used for relevant diagnostics of mercury allergy in man.

This rather unexpected finding could be explained if one could find crossreactivity to other organomercurials, which, according to published articles, can exist in oral cavity and in gastro-intestinal tract. One of these compounds is methylmercury. According to Heinze (Scand. J.Dental Res., 91:150-152, 1983), certain strains of oral microorganisms such as Streptococcus mitior, Streptococcus mutans and Streptococcus sanguis are able to transform metallic Hg and inorganic mercury salts into methyl-Hg. The same metabolic properties has been shown in certain bacterial strains which inhabit human gastrointestinal tract (Abdullah,M., Arnesjö B Einsi I., Scandinavian J. Gastroenterology, :565-567, 1973). Preliminary results indicate that lymphocytes from patients with oral problems whose lymphocytes react to phenyl mercury also crossreact with methylmercury salts such as methylmercuric chloride and also with ethylmercury compounds (Table 1, 9 and 13A). Positive lymphocyte responses against methyl-and ethy- mercurials were not found in healthy amalgam bearers or in amalgam-free donors (Table 1 and Table 14).

Taken together these results indicate that human lymphocyte reactivity against phenylmercury and other structurally related organomercurials can be used for an objective diagnosis in vitro of human mercury allergy. Thus, one can distinguish "real" mercury allergics from subjects who exhibit false-positive skin test and/or whose health problems are not caused by allergic reaction against mercury. Further on, one can determine if deviant response to mercurials can be found in patients with various autoimmune central nerve diseases such as multiple sclerosis (MS), amyelotrophic lateral sclerosis (ALS), Parkinsons disease, Alzheimer disease, acrodynia and others. Since many patients with oral lichen also suffer from other illnesses such as joint inflammation (Rheumatoid arthritis), diabetes, Sjögrens disease, Chronic Fatigue Syndrom or chronical fever, the potential relationship of these diseases with mercury allergy can be determined. If heavy metals, such as mercury or others act as causative or contributing agents in the development of these diseases, the universal chelating agents may be developed with the aim of better therapy.

Table 1

| Lymphocyte proliferation against organomercurials in patients 5 with oral lichen (L + S) and in healthy blood donors (SK). | | | | | | |
|---|---|---|---|---|---|---|
| Patient | Phenylmercuric Acetate | | | Metylmercuric Chloride | | |
| | CPM | SI | Lymphoblasts | CPM | SI | Lymphoblasts |
| L016 | 19,665 | 4.5 | + | 15,573 | 3.8 | + |
| S 32 | 6,434 | 4.3 | + | 2,527 | 2.3 | + |
| S 34 | 1,009 | 2.4 | + | 1,580 | 3.2 | + |
| S 93 | 3,294 | 2 | + | 22,819 | 4.3 | + |
| SK 45* | 424 | 2 | - | 439 | 1.3 | - |
| SK 46** | 140 | 1.1 | - | 871 | 1.8 | - |

* Healthy amalgam bearers
** Amalgam-free donors
SI = Stimulation Index

As shown in Figs 2 and 3, some of the healthy amalgam bearers and amalgam-free donors did not respond to any of the mercurial compounds studied. This can be explained by two ways: 1) certain persons exhibit genetical non responsiveness to mercury, 2) some people are desensibilized to mercurials through often life-long chronical exposition. The latter explanation seems to be supported by the fact that chronically exposed dental nurses often exhibit mercury-specific anergy (nonresponsiveness) at the memory cell level. Hence, inorganic mercury is not mitogenic for human lymphocytes as was untill now generally anticipated, but surprisingly, functions as an antigen. Reactivity to high concentrations of $HgCl_2$ (>0,5 micrograms per ml) could thus reflect immune reaction to inorganic mercury and, indirectly, mercury exposition.

Another distinguished feature of lymphocytes from some mercury-sensitized subjects is their ability to respond to low concentrations of inorganic $HgCl_2$ (< 0,5 micrograms per ml). Such cellular responses are found only in mercury-sensitized subjects and are absent in healthy subjects with or without amalgam. Thus, the presence of memory cells induced by minute amounts of inorganic mercury can be another feature characteristic for detection of mercury allergy in man.

Diagnosis of other metal allergies.

Several investigators demonstrated that metallic ions (corrosion products) from restorations, crowns, clasps, root fillings penetrate into the adjacent tissues such as dentin and enamel of the teeth (Söremark R, Wing K. Olsson K. Goldin J. Penetration of metallic ions from restorations into teeth. Journal Prost. Dent. 20,531-540.1968). The distribution of metallic ions from seemingly inert precious metals such as platinum or gold is potentiated by inflammatory processes in oral cavity. Thus, stimulated neutrophiles, monocytes and macrophages produce strongly oxidizing agents such as $H_2O_2$,OH and $O_2$ radicals in situ and contribute, together with oral microbial flora to generation of metal ions. Metal ions are taken up by immunological scavangers, monocytes and macrophages and distributed through the lymphoid and blood system through the entire body. Phagocytes containing metal content may be trapped in several organs such as joints or CNS and metal material released may stimulate lymphocytes present in the neighbourhood. Thus, this may be the reason for inflammatory processes observed often following the removal of metal fillings in patients with metal syndrom. This is also the reason behind the fact that metal-specific memory lymphocytes can be detected in the blood of subjects sensitized through the metal devices inserted into oral cavity or elsewhere in the body.

The new invention, MELISA, can now be used for analysis of immunological responses to wide variety of metals. The method can obviously be used also for the analysis of sensitizing potential of other metal implants inserted and chronically present elsewhere in the body (metal wires, pacemakers etc.). The following examples will demonstrate the usefulnes and the versatility of the new invention in this respect. All tests according to the invention were made with the procedure of example 2 below.

Platinum, gold and palladium.

These precious metals are used in so-called dental casting gold alloys (white gold) mostly for bridges but also for other restorations. Contact sensitization to gold crowns, bridges and denatures has been reported. Gold compounds have also been used in the treatment of rheumatoid arthritis (RA). Gold therapy has been associated with large number of adverse reactions. Several authors have attempted to use conventional previously named LTT to prove or disprove immunological nature of adverse reactions following chrysotherapy. However, the majority of reports describes just one or few patients studied and no individual dose responses for gold are given (Scapira D, Nahir M, Scharf y, Pollack S, Cholestatic jaundice induced by gold salts treatment. Clinical an immunological aspects-report of one case and review of the literature. J. Rheumatol. 11,843-845,1984, Evans, RB, Ettensson DB, Fawaz-Estrup F, Lally EV, Kaplan SR, Gold lung:recent development in pathogenesis, diagnosis and therapy, Seminars in arthritis and Rheumatism, 16,196-205, 1987). In one paper, immunological studies were performed on 12 patients with rheumatoid arthritis who developed reactions to gold. Two of 12 patients had positive LTT to gold (Davis P, Ezeoke, Munro J. Hobbs JR, Hughes GRV, Immunological studies on the mechanism of gold hypersensitivity reactions British Med. J. 3,676-678, 1973). No morphological evaluation was performed and low counts could be due to suboptimal method. Palladium is known to cause allergic contact stomatitis in patients with dental protheses. Such patients often report concominant patch reactions to nickel, cobalt and mercury (Hackel H, Miller K, Elsner P, Burg G, Unusual combined sensitization to palladium and other metals. Contact dermatitis, 24:131-132, 1991, Rebandel P, Rudzki E, Allergy to palladium. Contact Derm.23:121, 1990). Chloroplatinum salts are highly allergenic in occupational setting and exposition to platinum fumes give rise to platinum-specific IgE antibodies and asthma. Platinum-specific and palladium-specific memory lymphocytes have not been described previously.

Tin.

The sensitization properties of tin has not been studied in man. The use of this metal has increased during the last decade and represents high potential for pollution. In animals, stannic chloride and methyl tin trichloride increased antibody formation in mice (Dimitrov NV et al, Effect of tin on immune responses of mice, Clinical Immunol. and Immunopathol, 20:39-48, 1981). Tin is a standard constituent of amalgam alloys and penetration rate into enamel and dentin tissues is particularly high (von W Mocke, Untersuchungen durch Neutronenaktivierung über den diffundierten Elementgehalt von Zähnen mit Amalgamfüllungen, Deutsche Zahnärtzte, 26:657-664, 1971. No proliferative studies with tin with human lymphocytes have been detected in literature.

Titan.

Titan is generally considered non toxic and tissue compatible and have therefore been advocated as optimal metal material for dental restorations. It is widely used as an alternative to precious metals and together with baryum it forms an integral part of majority of gutta percha root fillings. However, the sensitizing properites of titanium in man have not been performed previously. In one case report, titan has been suggested as an aetiological agent in a case of granulomatous lung disease (Redline S, Barna BP, Tomashevski J, Abraham JL. Granulomatous diseas associated with pulmonary deposition of titanium. British J of Industrial Med. 43:652-65,1986). In one patient LTT to titanium chloride was performed and resulted in SI values 2 (weak borderline response) at one concentration only. No morphological studies were done. Due to these inconclusive results the possible sensitization potential of titan remained unsettled.

Copper and silver.

The allergies to copper and silver are rare. One single report describes the patient suffering from problems from silver amalgam restorations and exhibiting positive patch test to silver nitrate (Catsakis LH, Sulica VI, Allergy to silver amalgams. Oral medicine, 46:371-375, 1978). No proliferating studies with these metals were reported.

Nickel chromium and cobalt.

Chronic exposure to these metals is associated with several toxic and allergic effects including respiratory cancer due to chromium and nickel (Nieboer E, Sansford WE, Essencial toxic and therapeutic functions of metals including determinants of reactivity. Rev. Biochem Toxicol.7:205-245, 1985. At present time nickel is most common contact sensitizer with prevalence rating about 15% in Swedish female population. Patch tests are routinely used for supporting of clinical history of nickel, chrome and cobalt allergy. Under certain circumstances, false-negative results during patch testing may compromize allergen identification (Keczkes K, Basheer AM, Wyatt EH, The persistence of allergy contact sensitivity: a 10 year follow up in 100 patients. Bitish J. Dermatol. 107-461-465, 1982, Dooms-Gossen SA Vanmaele N. Degreef H, Follow up study of patients with contact dermatitis caused by chromates nickel and cobalt. Dermatologica, 160:249-260, Mendelow AJ, Forsyth A, Florence AT, Baiillie AJ, Patch testing for nickel allergy. The influence of the vehicle on the response to topical nickel sulfate. Contact dermatitis 3:29-33, 1985.

Metal devices used in dentistry and orthopaedic surgery are usually made of stainless steel, containing both chromium and nickel. Adverse reactions from such implants are considered to be rare but may occur (Schiver WR, Shereff, Domnitz JM, Swintak EF and Civjan SC, Allergic response to stainless steel wire, Oral Surg. 42:578-581, 1976.

Lymphocyte transformation test (LTT) has been attempted for the diagnosis of contact dermatitis due to nickel, cobalt and chromium (Al Tawil NG, Immunological studies in patients with nickel, cobalt and chromium sensitivity, Doctoral thesis, Stockholm, 1985). However, while it was possible to distinguish between group of patients and controls, there was an overlap among the two groups and therefore the value of the test for the individual, patient remained low (Veien NK, Diagnostic procedures for eczema patients. In: Exogenous dermatoses:environmental dermatitis, T. Menné and HI Maibach (Eds) CRC Press, 1991.

New invention, MELISA, can be used for in vitro diagnosis of allergy to nickel and cobalt (patient S 85) and of chromium (patient VM 16, Table 11).

Cadmium, Lead and arsenic.

The influence of lead, cadmium and arsenic on the immune system is still poorly established. A number of studies suggest that the immune competence of exposed animals may be significantly altered (Descotes J, Verdier F, Brouland JP, Pulce C, Immunotoxicity of lead, cadmium and arsenic. Experimental data and their relevance to man. In: Immunotoxicity of metals and immunotoxicology. Dayan AD, Hertel RF, Heseltine E, Kazantzis G, Smith EM van der Venne MT, Eds.j Plenum press, NY and London, 1990.

No published data were found on proliferative responses of human memory lymphocytes to these metals.

Patient tests.

VM 39, female nurse, has been suffering from chronical depression in combination with metal syndrom. She suspected mercury in amalgam fillings as a cause of her illness. She also had some gold fillings in her oral cavity. Amalgam fillings were removed and gold-platinum bridge has been inserted. However, no improvement of patient health was noted. The patient has suspected residual mercury present elsewhere in the body and asked for analysis of metals in liquor. The analysis revealed negligible concentrations of mercury ions but high concentrations of platinum ions. Patient's lymphocytes responded to platinum, palladium and gold, components of gold alloy bridge (Table 2). Her lymphocytes also responded to tin (a component of amalgam) but not to mercurials, copper, silver and titanium salts. The patient's symptoms gradually improved following the removal of dental metals.

VM 40, a 26 year old male, suffered form fibromyalgia and chronic fatigue syndrom for 10 years. The symptoms debuted following the insertion of gold bridge. MELISA results (Table 3) indicate lymphocyte sensitization to platinum, palladium, titanium and tin.

VM 35, female journalist, suffered from metal syndrom and depression periods for considerable length of time. She suspected metal dental material as a cause of her illness. She had several root fillings containing gutta percha and gold and amalgam restorative material in oral cavity. MELISA (Table 4) shows lymphocyte proliferation to titan but not to other metals tested such as mercurials, gold and platina. Thus, this patient's problems may be caused by titan-containing root fillings but not by other metal restorations.

S 85, 55 years old female, suffered from metal syndrom and oral burning and itching in connection with insertion of gold bridge. Previously she noticed intolerance to nickel which was confirmed by positive patch test (Table 5). In MELISA, lymphocytes responded to nickel, cobalt, inorganic and organic gold and to lead. She did not react to chromium, copper and mercurials. A new patch test was performed with gold thiosulfate and cobalt chloride. The patch reaction was positive to cobalt but negative to gold despite of strong reactivity detected in MELISA. This again indicates the advantages of MELISA in the diagnosis of metal allergies.

SK 1, a 46 years old male physician, suffered from multiple sclerosis for several years. His illness diminished and gradually disappeared following the removal of root-filled teeth ($N_2$, containing lead and phenyl mercury) and of gold plug. MELISA indicated strong lymnphocyte proliferation to gold and weak response to phenylmercuric acetate (Table 6).

Occasionally, proliferative responses to high concentrations of gold thiosulfate (10-50 micrograms per ml) were observed in subjects who did not experience any problems related to gold material. Human population is widely exposed to gold through jewelry and such responses could indicate exposure to gold rather than allergy to this particular metal. On the contrary, persons with clinical symptoms relevant to gold devices such as patients SK 1 and S 85 responded in MELISA to much lower concentrations of gold (less than 10 micrograms of gold per m1). Thus, arbitrary, positive lymphocyte responses to low contrations of gold thiosulfate were taken as an evidence of gold allergy. In such patients, gold-specific patch tests were occasionally demonstrated (SK 1). AD 4 and AD 6 are another two female patients suspecting metal restorations in the oral cavity as a cause of their chronic fatigue and malaise. Several precious metals were used to restore teeth in the past together with many root fillings. MELISA (Tables 7 and 8) shows vigorous proliferation to gold thiosulfate and weak response to silver acetate in patient AD 4 and positive proliferation to gold, palladium, phenyl mercury and silver in patient AD 6.

Table 9 shows MELISA performed with lymphocytes of 28 years old female with no metal restorations and no root fillings in the oral cavity. Donor 312 was not exposed to gold and titan (Table 10). The MELISA results in these unexposed persons were invariably negative.

A 46 year old female VM 16, Table 11 suffered from Quinques oedema localized around the eyes which debuted following insertion of dental device made of stainless steel. Previously, she experienced itching and burning sensation in the vicinity of her gold and amalgam fillings. She also suffered from chronical headaches. Patient's lymphocytes reacted strongly to nickel and chromium salts and also to gold and organic mercury. Lymphocytes did not respond to cobalt. Patch test with cobalt was performed twice in 3 months interval. Patch test was positive with 1% solution of cobalt chloride but negative to 0.5% solution. This indicates toxic rather than allergic reaction of the skin to cobalt. Patch test with mercurials were negative in spite of positive MELISA.

Proliferation to cadmium is illustrated in patient VM 45 (Table 12) and to lead in patient S 85 (Table 5).

The representative data shown above indicate the vast potential of MELISA to measure allergy to virtually all metals present chronically in odontological materials or in another medical metal devices. In future, this technique will also be used to evaluate possible allergenic properties of non metallic odontological materials. As mentioned previously, the screening for deviant reactions to various metals in systemic diseases will give us a clue not only to diagnosis but also to the prevention and better treatment of illnesses depending on metal ethiology.

The process according to the invention will now be illustrated with the help of examples.

Example 1

Cultivation in Tubes

50 ml blood was diluted with 50 ml of RPMI 1640 medium and diluted blood was layered over Ficoll-Paque gradient in 2:3 ratio in 50 ml sterile plastic tubes (Becton Dickinson) and was separated by gradient centrufugation at 600 g for 30 minutes. The interphase (opaque white layer) containing mainly lymphocytes and monocytes was removed and washed once with RPMI 1640 medium.

Cells were then resuspended in RPMI 1640 medium containing 20% heat-inactivated human AB + serum, glutamine and gentamycin and were then transferred to a plastic tissue culture bottle. The tissue culture bottle was placed into $CO_2$-incubator gassed with 5% $CO_2$ in the air and incubated for 2 hours. Monocytes and other adherent cells adhered to the plastic surface and were retained in the plastic bottle. Non adherent cells were removed and diluted twice with RPMI 1640 medium. Cells were counted by removing of x microliter of cells and staining them with Turk. The number of cells was determined by counting in Burker chamber under the microscope. Finally, the cells were diluted to the concentration of 0.5 x $10^6$ cells per ml.

2 ml aliquots of lymphocyte suspension containing 10% heat inactivated AB + were added into 10 ml round bottomed sterile tubes which contained 100 microliters of dilution containing various concentrations of $HgCl_2$ or phenylmercuric acetate. Tubes were cultured in $CO_2$ incubator and 100 % humidity for 6 days.

10 microliter of methyl-$^3$H-thymidin (37 kBq per well), specific activity 3.18 TBq/mmol was distributed to each of 96 wells in plastic plate. 200 microliters of cell suspension in triplicates was then transferred from each macroculture. The plates were incubated at 37°C for 4 hours in $CO_2$ incubator. After incubation cells were harvested in an automatic cell harvester (INOTECH). Distilled water was used for washing. Plates were harvested immediately.

Filter discs containing radioisotope-labeled content of lysed cells were transferred into microampoulles and 1 ml of Opti-Scint (LKB) was added. Ampoulles were closed and radioactivity was measured in Beta scintillation counter. The results are depicted in Fig.4.

Another sample was taken from each tube for morphological evaluation under the microscope. 75 microliters of cell suspension was taken from each culture and was centrifuged in cytocentrifuge at 90g for 2 minutes. Cell smears were fixed in methanol and were further stained with May Grunewald and Giemsa. The results are shown in Table 13. It can be seen that patient 31 exhibits positive results with $HgCl_2$ and with phenylmercuric acetate in MELISA. These results indicate the presence of mercury allergy.

Table 13

| Patient S31-0ral lichen-morphological evaluation. | | |
|---|---|---|
| Antigen | Microgram/ ml | Lymphoblasts/prep |
| Control | - | 1 , 0 |
| HgCl$_2$ | 1<br>0,5<br>0,25<br>0,12<br>0,06 | 23<br>100<br>>100<br>>100<br>>100 |
| Phenylmercury acetate | 1<br>0,5<br>0,25<br>0,12<br>0,06 | - toxic<br>7<br>>100<br>53<br>39 |
| Tuberculin(PPD) | 1 | >100 |
| Evaluation:<br>1. Positive response to all concentrations of HgCl$_2$<br>2. Positive response to phenyl mercury<br>3. MELISA results show mercury allergy. | | |

Example 2

Cultivation on 48-well plate.

50 ml blood was diluted with 50 ml of RPMI 1640 medium and was layered over Ficoll-Isopaque (Pharmacia) in sterile centrifugation tubes made of plastics. The blood was centrifuged for 30 minutes at 600 g as in Example 1.

After centrifugation, the white opaque interphase containing mainly lymphocytes and monocytes was separated and washed once with RPMI 1640 medium containing 20% heat inactivated human AB + serum, glutamine and gentamycine. The cell suspension was transferred into tissue culture bottle which was placed into C0$_2$ incubator containing 5% C0$_2$ in the air. Cells were incubated at 100% humidity at 37°C for 1 hour. During this period monocytes and other adherent cells attached to the plastics and could easily be separated from non adherent lymphocyte fraction. The lymphocyte fraction was diluted twice with RPMI 1640 medium. Cell counting was performed as in Example 1. The cells were diluted to concentration 1 x 10$^6$ per ml. 1 ml of lymphocyte suspension in RPMI 1640 containing 10 % heat inactivated AB + serum, glutamine and gentamycine were added to each of the 48 wells. In each well there were already 100 microliter of various dilutions of mercurials. The cultures were incubated for 5 days in the C0$_2$ incubator as described in Example 1.

After 5 days of cultivation, the content of each well was resuspended 3 times. 600 microliters from each culture were removed and transferred to a new 48-well plate. In this radioactive plate, each well contained 10 microliters of methyl-$^3$H-thymidine with specific activity 3.18 Tbq/mmol, (111 kBq per well). Incubation proceeded for 4 hours at 37°C in C0$_2$ incubator. After incubation, cells were harvested in fully automatic cell harvester as in Example 1. Filter papers containing lysed cell filtrate were placed in microampoulles and 1 ml of Opti-Scint(LKB) was added. The ampoules were closed and the radioactivity was recorded on beta scintillation counter. The following results were obtained and are presented in Table 14A,B and in Table 15.

It shows lymphocyte proliferation against various mercury compounds presented as radioactive counts and as morphological evaluation.

Patient L16 (Table 14A) shows positive response to all 3 mercury compounds tested. The results thus indicate mercury allergy.

Patient S 92 (Table 14B) shows, on the contrary, positive response to high dosis of HgCl$_2$ only, but not to low doses of inorganic mercury or to phenylmercuric acetate. Thus, these results do not indicate mercury allergy.

Table 15 shows lymphocyte proliferation against different mercury compounds in healthy amalgam bearer. The results are expressed as isotope incorporation into DNA (counts per minute) and by morphological evaluation. As can be seen, MELISA performed with high doses of $HgCl_2$ was positive (2.25 micrograms and 4.5 micrograms per ml). However, the patient's lymphocytes did not respond to other mercurials such as phenyl-, ethyl-, or methylmercuric chloride. This pattern of reactivity is found in healthy persons and therefore does not indicate allergy.

The present process is very useful for in vitro analysis of allergies induced by non endogenic antigenic agents such as metals, metal containing compounds, plastic dental fillings. One great advantage is that it can be performed in vitro and that consequently the patient need not to be exposed by the antigen for testing purpose. The process can also be used to test other non metallic materials intended for use in implantates before they are used to a greater extent.

## Table 2

### LYMPHOCYTE PROLIFERATION TO METALS IN DONOR VM 39

| Lymphocyte treatment | Concentration μg/ml | Λcpm | SI | Number of lymphoblasts |
|---|---|---|---|---|
| Control | - | (2715+948)* | 1.0 | 2, 4, 4 |
| PPD | 1.0 | 1220 | 1.4 | 0 |
| $HgCl_2$ | 0.5 | 1634 | 1.6 | 0 |
| | 0.25 | 2041 | 1.8 | 34 |
| | 0.125 | -666 | 0.8 | 9 |
| Phenylmercuric chloride | 1.0 | -1632 | 0.6 | 7 |
| | 0.5 | -1488 | 0.8 | 4 |
| | 0.25 | -1209 | 0.6 | 3 |
| Methylmercuric chloride | 1.0 | -470 | 0.8 | 2 |
| | 0.5 | -1027 | 0.6 | 10 |
| | 0.25 | 77 | 1.0 | 9 |
| Goldsodium thiosulfate | 5 | 3580 | 2.3 | 37 |
| | 2.5 | 796 | 1.3 | 14 |
| | 1.25 | 948 | 1.3 | 14 |
| Platinum sulfate | 100 | 3921 | 2.4 | 0 |
| | 50 | 12034 | 5.4 | 28 |
| | 25 | 11050 | 5.1 | 11 |
| Stannous chloride (Tin) | 200 | -2199 | 0.2 | + |
| | 100 | 5249 | 2.9 | 11 |
| | 50 | 4228 | 2.6 | 48 |
| Copper sulfate | 1 | -1377 | 0.5 | 0 |
| | 0.5 | -1515 | 0.4 | 4 |
| | 0.25 | -1486 | 0.4 | 5 |
| Silver nitrate | 10 | -851 | 0.7 | 7 |
| | 5 | -1642 | 0.4 | 0 |
| | 2.5 | 192 | 1.1 | 1 |
| Titan sulfate | 10 | -434 | 0.8 | 1 |
| | 5 | 418 | 1.2 | 6 |
| | 2.5 | 819 | 1.3 | 23 |

Table 2 (Cont.)

| Lymphocyte treatment | Concentration µg/ml | Δcpm | SI | Number of lymphoblasts |
|---|---|---|---|---|
| Palladium chloride | 50 | 14237 | 6.2 | 43 |
| | 25 | 9792 | 4.6 | 30 |
| | 12.5 | 1308 | 1.5 | 20 |
| Thimerosal | 1 | −1476 | 0.4 | 5 |
| | 0.5 | 345 | 1.1 | 4 |
| | 0.25 | 889 | 1.3 | 8 |

EVALUATION:

Patient VM 39 is positive to: Gold
                              Platina
                              Tin
                              Palladium

                negative to: $HgCl_2$
                             Phenylmercuric acetate
                             Methylmercuric chloride
                             Copper
                             Silver
                             Titan
                             Thimerosal

Table 3

LYMPHOCYTE PROLIFERATION TO VARIOUS METALS IN DONOR VM 40

| Lymphocyte treatment | Concentration µg/ml | Λcpm | SI | Number of lymphoblasts |
|---|---|---|---|---|
| Control | - | (884+45)* | 1.0 | 1, 0, 0 |
| PPD | 1.0 | 335172 | 380 | >100 |
| HgCl$_2$ | 0.5 | 1036 | 2.1 | 0 |
| | 0.25 | 287 | 1.3 | 0 |
| | 0.125 | 974 | 2.1 | 3 |
| Phenylmercuric chloride | 1.0 | -171 | 0.8 | 0 |
| | 0.5 | 752 | 1.8 | 1 |
| | 0.25 | 257 | 1.3 | 0 |
| Methylmercuric chloride | 1.0 | 1032 | 2.2 | 4 |
| | 0.5 | -201 | 0.8 | 2 |
| | 0.25 | -108 | 0.9 | 2 |
| Goldsodium thiosulfate | 5 | 406 | 1.5 | 3 |
| | 2.5 | 567 | 1.6 | 3 |
| | 1.25 | 774 | 1.9 | 1 |
| Platinum sulfate | 100 | 1040 | 2.2 | 2 |
| | 50 | 1969 | 3.2 | 7 |
| | 25 | 1670 | 2.9 | 2 |
| Stannous chloride (Tin) | 200 | 962 | 2.1 | + |
| | 100 | 8098 | 10 | 17 |
| | 50 | 9494 | 12 | 66 |
| Copper sulfate | 1 | -280 | 0.7 | 0 |
| | 0.5 | -350 | 0.6 | 3 |
| | 0.25 | -534 | 0.4 | BS |
| Silver nitrate | 10 | -102 | 0.9 | 4 |
| | 5 | -191 | 0.8 | BS |
| | 2.5 | -166 | 1.8 | 4 |
| Titan sulfate | 10 | 1646 | 2.8 | 4 |
| | 5 | 1240 | 2.4 | 46 |
| | 2.5 | -134 | 0.8 | 0 |

Table 3 (Cont.)

| Lymphocyte treatment | Concentration µg/ml | Δcpm | SI | Number of lymphoblasts |
|---|---|---|---|---|
| Palladium chloride | 50 | 30710 | 36 | 3 |
| | 25 | 19448 | 23 | 21 |
| | 12.5 | 3188 | 4.6 | 10 |
| Thimerosal | 1 | -328 | 0.6 | 0 |
| | 0.5 | -217 | 0.8 | 1 |
| | 0.25 | 522 | 1.6 | 2 |

EVALUATION:

Patient VM 40 is positive to: Palladium
Titan
Tin

weakly positive to: Platina

negative to: $HgCl_2$
Phenylmercuric acetate
Methylmercuric chloride
Copper
Silver
Thimerosal
Gold

Table 4

RESULTS OF  M E L I S A  IN PATIENT NO: VM 35

LYMPHOCYTE PROLIFERATION TO PPD, MERCURY, PHENYLMERCURY, GOLD, PLATINUM, AND TITANIUM IN SUBJECT NO. VM 35

| Lymphocyte treatment | Concentration µg/ml | Δcpm | SI | Number of lymphoblasts |
|---|---|---|---|---|
| RPMI medium | – | (3022+1838)* | 1.0 | 8 |
| | – | | | 0 |
| | – | | | 2 |
| | – | | | |
| | – | | | |
| | – | | | |
| Positive control PPD | 1.0 | 675586.3 | 224.6 | >100 |
| Test substances: | | | | |
| 1. HgCl$_2$ | 1.0 | −586.5 | 0.8 | 0  A MF |
| | 0.5 | −1090.6 | 0.6 | 0 |
| | 0.25 | −1169.7 | 0.6 | 3 |
| | 0.125 | −956.3 | 0.7 | 0 |
| 2. Phenylmercuric acetate | 1.0 | −2733.6 | 0.1 | –  toxic |
| | 0.5 | −1496.4 | 0.5 | 1 |
| | 0.25 | 826.2 | 1.3 | 0 |
| | 0.125 | 1125.1 | 1.4 | 2 |
| 3. Goldsodium thiosulfate | 12.5 | −1364.1 | 0.5 | 0 |
| | 6.25 | −886.2 | 0.7 | 0 |
| | 3.12 | −878.2 | 0.7 | 0 |
| | 1.56 | −1075.2 | 0.6 | 1 |
| 4. Platinium sulfate | 125.0 | −2713.3 | 0.1 | –  toxic |
| | 62.5 | −460.2 | 0.8 | 5 |
| | 31.25 | 2666.6 | 1.9 | 9 |
| | 15.6 | −324.9 | 0.9 | 1 |
| | 7.8 | −1325.0 | 0.6 | 2 |
| | 3.9 | −1382.1 | 0.5 | 0 |

Table 4 (Cont.)

| Lymphocyte treatment | Concentration µg/ml | Δcpm | SI | Number of lymphoblasts |
|---|---|---|---|---|
| 5. Titanium | 50.0 | -2083.1 | 0.3 | - toxic |
| (III) | 10.0 | -592.5 | 0.8 | 1 |
| sulfate | 5.0 | 5084.1 | 2.7 | 18 |
| | 2.5 | 23207.0 | 8.7 | 50 |

Footnote

Δcpm = cpm in test culture minus mean cpm in negative control cultures
* = mean cpm ± standard deviation
SI = stimulation index = cpm in test culture divided by mean cpm in control cultures

PPD = tuberculin purified protein derivative
ND = not done
(+) = partly toxic
A MF = activated macrophages

EVALUATION: 1. Positive proliferation to titaniumsulfate

2. Negative to mercurials

3. Negative to gold and platinium.

18

Table 5

LYMPHOCYTE PROLIFERATION TO VARIOUS METALS IN DONOR S 85

| Antigen in culture | Concentration µg/ml | $\Delta$cpm | SI | Number of lymphoblasts |
|---|---|---|---|---|
| Control | - | (1419$\pm$180)* | 1.0 | 1, 0, 8<br>6, 13, 5 |
| PPD | 1.0 | 384313 | 271.8 | >100 |
| $NiCl_2$ ** | 20.0 | 188638 | 133.9 | >100 |
| | 10.0 | 163936 | 116.5 | >100 |
| | 5.0 | 99092 | 70.8 | >100 |
| | 2.5 | 98735 | 70.6 | >100 |
| | 1.25 | 32652 | 24.0 | >100 |
| | 0.62 | 43786 | 31.9 | >100 |
| $CrCl_3$ * | 20.0 | -293 | 0.8 | 2 |
| | 10.0 | 686 | 1.5 | 2 |
| | 5.0 | -488 | 0.7 | 7 |
| | 2.5 | 1561 | 2.1 | 1 |
| | 1.25 | -277 | 0.8 | 3 |
| | 0.62 | -164 | 0.9 | 2 |
| $CoCl_2$ ## | 20.0 | 745 | 1.5 | 24 |
| | 10.0 | 1035 | 1.7 | 42 |
| | 5.0 | 11083 | 8.8 | 80 |
| | 2.5 | 7592 | 6.3 | 30 |
| | 1.25 | 6486 | 5.6 | 50 |
| | 0.62 | 6553 | 5.6 | 32 |
| $HgCl_2$ # | 4.0 | 31405 | 23.1 | 70 |
| | 2.0 | 3620 | 3.6 | 10 |
| | 1.0 | -589 | 0.6 | 2 |
| | 0.5 | 650 | 1.5 | 2 |
| | 0.25 | -6 | 1.0 | 0 |
| | 0.125 | -337 | 0.8 | 0 |

Table 5 (Cont.)

LYMPHOCYTE PROLIFERATION TO VARIOUS METALS IN DONOR S 85 (cont.)

| Antigen in culture | Concentration μg/ml | Λ cpm | SI | Number of lymphoblasts | |
|---|---|---|---|---|---|
| Goldsodium # thiosulfate | 50.0 | 2973 | 3.1 | – | T |
| | 25.0 | 32223 | 23.7 | 72 | |
| | 12.5 | 3774 | 3.7 | >100 | |
| | 6.25 | 5234 | 4.7 | 50 | |
| | 3.12 | 3506 | 3.5 | >100 | |
| Auranofin | 4.0 | –1076 | 0.2 | – | T |
| | 2.0 | –1054 | 0.3 | – | T |
| | 1.0 | 9591 | 7.8 | 38 | |
| $CuSO_4$ | 1.0 | –753 | 0.5 | 6 | |
| | 0.5 | –921 | 0.4 | 13 | |
| | 0.25 | –831 | 0.4 | 1 | |
| | 0.125 | –730 | 0.5 | 5 | |
| | 0.062 | –796 | 0.4 | 0 | |
| | 0.031 | –646 | 0.5 | 8 | |
| $Pb(NO_3)_2$ | 50.0 | 657 | 1.5 | – | T |
| | 25.0 | 3305 | 3.3 | 1 | |
| | 12.5 | 2046 | 2.4 | 40 | |
| | 6.25 | 2699 | 2.9 | 18 | |
| | 3.12 | 1313 | 1.9 | 14 | |
| | 1.56 | 685 | 1.5 | 2 | |

PPD        Tuberculin, Purified Protein Derivative
*          mean cpm ± standard deviation
T          toxic
##         Patch-test positive
#          Patch-test negative

EVALUATION: 1) Very strong positive proliferation to Nickel.

2) Positive to Cobalt, inorganic Gold, Auranofin (organic gold) and Lead.

3) Negative to Chromium and Copper

4) Response to inorganic Mercury in the range of normal.

Table 6

| LYMPHOCYTE PROLIFERATION TO VARIOUS METALS IN DONOR SK 1 | | | | | |
|---|---|---|---|---|---|
| Antigen in culture | Concentration μg/ml | Λcpm | SI | Number of lymphoblasts | |
| Control | - | (1631±466)* | 1.0 | 1, 0, 5 5, 0, 4 | |
| PPD | 1.0 | 842574 | 517.5 | >100 | |
| Goldsodium ## thiosulfate | 50.0 25.0 12.5 6.25 3.12 1.56 | 16681 48828 10719 20903 5234 8852 | 11.2 30.9 7.6 13.8 4.2 6.4 | >100 >100 >100 >100 >100 60 | PT |
| Phenylmercuric # acetate | 1.0 0.5 0.25 0.125 0.062 0.031 | -1444 -631 2078 2678 1153 189 | 0.1 0.6 2.3 2.6 1.7 1.1 | - 8 25 0 0 0 | T |
| Thimerosal # | 1.0 0.5 0.25 0.125 0.062 | -1550 -517 1183 420 583 | <0.1 0.7 1.7 1.3 1.4 | - 19 10 4 3 | T |
| PPD Tuberculin, Purified Protein Derivative * mean cpm ± standard deviation T toxic PT partly toxic ## Patch-test positive # Patch-test negative EVALUATION: 1) Strong proliferative response to Goldsodium thiosulfate, indicating Gold allergy 2) Weak proliferation to Phenylmercury 3) Negative to Thimerosal | | | | | |

Table 7

LYMPHOCYTE PROLIFERATION TO VARIOUS METALS IN DONOR AD 4

| Antigen in culture | | Concentration µg/ml | Λcpm | SI | Number of lymphoblasts |
|---|---|---|---|---|---|
| Control | | | (2458±517) | 1.0 | 1, 9, 3, 3, 5, 6 |
| PPD | | 1.0 | 342836 | 140.4 | >100 |
| $HgCl_2$ | ♯ | 4.0 | 70029 | 29.5 | 70 |
| | | 2.0 | 20166 | 9.2 | 50 |
| | | 1.0 | 3660 | 2.5 | 10 |
| | | 0.5 | 750 | 1.3 | ND |
| | | 0.25 | 1434 | 1.6 | ND |
| | | 0.12 | −526 | 0.8 | ND |
| Phenylmercuric acetate | ♯ | 1.0 | −1909 | 0.2 | ND |
| | | 0.5 | 1953 | 1.8 | 7 |
| | | 0.25 | −512 | 0.8 | ND |
| | | 0.125 | −608 | 0.7 | ND |
| | | 0.06 | −904 | 0.6 | ND |
| Goldsodium thiosulfate | ♯♯ | 25.0 | 94221 | 39.3 | 13 |
| | | 12.5 | 99659 | 41.5 | 90 |
| | | 6.2 | 49924 | 21.3 | >100 |
| | | 3.1 | 40018 | 17.3 | >100 |
| | | 1.5 | 46407 | 19.9 | >100 |
| | | 0.7 | 31461 | 13.8 | >100 |
| Silver acetate | ♯♯ | 50.0 | −2349 | 0.04 | ND |
| | | 25.0 | −2347 | 0.04 | ND |
| | | 12.5 | −2348 | 0.04 | G  + |
| | | 6.2 | 544 | 1.2 | 10 |
| | | 3.1 | 6609 | 3.7 | 36 |
| | | 1.5 | −45 | 0.9 | 0 |

ND    not done
G    granulocytes
+    toxic
♯♯    Patch-test positive
♯    Patch-test negative

EVALUATION: 1. Vigorous proliferative response to Gold thiosulfate.
2. Weak proliferation to Silver acetate.
3. Positive to highest concentrations of $HgCl_2$ in the range of normal responses
4. Negative to phenyl-Hg

Table 8

AB ASTRA                                                          1
Safety Assessment/RS
Immunotoxicology

LYMPHOCYTE PROLIFERATION TO VARIOUS METALS IN DONOR AD 6

| Lymphocyte treatment | Concentration μg/ml | Δcpm | SI | Number of lymphoblasts |
|---|---|---|---|---|
| Control | | (1378±385) | 1.0 | 0, 0, 0, 0, 0, 0 part. lysed |
| PPD | 1.0 | 201190 | 146.9 | >100 |
| HgCl$_2$    # | 4.0 | 106284 | 78.0 | ND |
| | 2.0 | 30113 | 22.8 | ND |
| | 1.0 | 7958 | 6.7 | ND |
| | 0.5 | 1464 | 2.0 | ND |
| | 0.25 | 396 | 1.3 | ND |
| | 0.12 | 160 | 1.1 | ND |
| | 0.06 | 583 | 1.4 | |
| Phenylmercuric acetate | 1.0 | −1137 | 0.1 | ND |
| | 0.5 | 6846 | 5.9 | 10 |
| | 0.25 | 5375 | 4.9 | 28/27 |
| | 0.12 | 2342 | 2.7 | ND |
| | 0.06 | 543 | 1.4 | ND |
| | 0.03 | −481 | 0.6 | |
| Thimerosal | 1.0 | −865 | 0.3 | ND |
| | 0.5 | −453 | 0.7 | ND |
| | 0.25 | −515 | 0.6 | ND |
| | 0.12 | −641 | 0.5 | ND |
| | 0.06 | −377 | 0.7 | |
| Goldsodium thiosulfate    ## | 10.0 | 32108 | 24.3 | >100 |
| | 5.0 | 25392 | 19.4 | >100 |
| | 2.5 | 13154 | 10.5 | 20 |
| Palladium chloride | 50.0 | 53379 | 39.7 | ND |
| | 25.0 | 7502 | 6.4 | ND |
| | 12.5 | −499 | 0.6 | ND |
| | 6.25 | −365 | 0.7 | ND |
| | 3.1 | −472 | 0.6 | ND |
| | 1.5 | −599 | 0.6 | |

Table 8 (cont.)

LYMPHOCYTE PROLIFERATION TO VARIOUS METALS IN DONOR AD 6 (cont.)

| Antigen in culture | Concentration µg/ml | Λcpm | SI | Number of lymphoblasts |
|---|---|---|---|---|
| Silver acetate | 50.0 | -1291 | 0.06 | ND |
| | 25.0 | -1313 | 0.04 | ND |
| | 12.5 | -1321 | 0.04 | ND |
| | 6.25 | 272 | 1.1 | ND |
| | 3.1 | 2231 | 2.6 | 28 |
| | 1.5 | 14 | 1.0 | ND |
| | 0.7 | 1075 | 1.8 | ND |

ND   not done
##   Patch-positive

EVALUATION: 1. Positive proliferation to high concentrations of
               $HgCl_2$ in the range of normal responses.
            2. Positive proliferation to Phenylmercuric and
               Palladium
            3. Strong positive proliferation to low concentrations
               of Gold.
            4. Weak proliferation to Silver
            5. This patent indicates deviant response to organic
               Mercury, inorganic Gold, Palladium and Silver.

24

Table 9

| LYMPHOCYTE PROLIFERATION IN DONOR TL 80 | | | | |
|---|---|---|---|---|
| Antigen in culture | Concentration µg/ml | Λcpm | SI | Number of lymphoblasts |
| Control | - | (3977±1346)* | 1.0 | -, 0, 4, 0, 3, 1 |
| PPD | 1.0 | 479590.7 | 121.6 | >100 |
| Test substances: | | | | |
| Phenylmercuric acetate | 1.0 | -1197.8 | 0.7 | - toxic |
| | 0.5 | -2538.6 | 0.4 | 0 |
| | 0.25 | -508.0 | 0.9 | 2 |
| | 0.125 | -415.5 | 0.9 | 0 |
| Methylmercuric chloride | 1.0 | 1971.8 | 1.5 | - toxic |
| | 0.5 | -2630.9 | 0.3 | 1 |
| | 0.25 | -1486.5 | 0.6 | 0 |
| | 0.125 | -1956.5 | 0.5 | 0 |
| Thimerosal | 1.0 | -83.8 | 1.0 | - toxic |
| | 0.5 | -3516.2 | 0.1 | 0 PT |
| | 0.25 | -3294.9 | 0.2 | 0 |
| | 0.125 | -2389.8 | 0.4 | 0 |
| | 0.062 | -147.0 | 1.0 | 8 |
| Goldsodium thiosulfate | 50.0 | -664.4 | 0.8 | 5 grains |
| | 25.0 | -5.5 | 1.0 | 1 |
| | 12.5 | -1554.6 | 0.6 | 0 |
| | 6.25 | -1219.9 | 0.7 | 5 |
| | 3.12 | -2085.8 | 0.5 | 0 |
| | 1.56 | -2745.9 | 0.3 | 0 |
| Platinum | 125.0 | -1153.6 | 0.7 | 0 |
| | 62.5 | -2673.9 | 0.3 | 0 ** |
| | 31.25 | -1751.1 | 0.6 | 0 |
| | 15.6 | -1110.6 | 0.7 | 0 |
| | 7.8 | -2075.8 | 0.5 | - ND |
| PPD = tuberculin purified protein derivative<br>ND = not done<br>PT = partly toxic<br>** = activated macrophages containing metal debris<br>EVALUATION: Negative to Phenylmercury, Methylmercury, Thimerosal, Gold and Platinum. | | | | |

Table 10

| LYMPHOCYTE PROLIFERATION TO VARIOUS METALS IN DONOR 312 | | | | |
|---|---|---|---|---|
| Antigen in culture | Concentration $\mu$g/ml | cpm±SD | SI | Number of lymphoblasts |
| Control<br>PPD | -<br>0.1 | 1322±285<br>419837 | 1<br>318 | 0<br>>100 |
| Pb (NO$_3$)$_2$ | 50<br>25<br>12.5<br>6.25<br>3.12<br>1.56 | 723<br>536<br>716<br>718<br>2099<br>1658 | 0.5<br>0.4<br>0.5<br>0.5<br>1.6<br>1.3 | 1<br>2<br>2<br>1<br>0<br>1 |
| Gold sodium thiosulfate | 50<br>25<br>12.5<br>6.25<br>3.12<br>1.56 | 4041<br>3007<br>1039<br>1679<br>2634<br>1698 | 3.1<br>2.3<br>0.8<br>1.3<br>2.0<br>1.3 | 0<br>0<br>0<br>0<br>0<br>0 |
| Titan sulfate | 100<br>50<br>25<br>12.5<br>6.25<br>3.12<br>1.56<br>0.8 | 2294<br>1293<br>881<br>931<br>1027<br>2126<br>1163<br>1327 | 1.7<br>1.0<br>0.7<br>0.7<br>0.8<br>1.6<br>0.9<br>1.0 | 1[a]<br>7<br>0<br>0<br>0<br>0<br>0<br>0 |
| [a] crystals, partly toxic<br>Evaluation: Negative to lead, gold and titanium salts | | | | |

Table 11

LYMPHOCYTE PROLIFERATION TO PPD, $HgCl_2$, PHENYLMERCURIC ACETATE, $CoCl_2$, $NiCl_2$, $CrCl_3$ AND GOLD SODIUM THIOSULPHATE IN SUBJECT NO. VM 16

| Antigen in culture | Concentration µg/ml | Λcpm | SI | Number of lymphoblasts | |
|---|---|---|---|---|---|
| Negative control | - | $(2316\pm640)*$ | 1.0 | 1 | |
| | - | | | 2 | |
| | - | | | 4 | |
| | - | | | 10 | |
| | - | | | ND | |
| | - | | | ND | |
| PPD | 1.0 | 290392 | 126.4 | >100 | |
| Test substances | | | | | |
| 1. $HgCl_2$£ | 4.0 | 21643 | 10.3 | 21 | MF |
| | 2.0 | 11360 | 5.9 | 8 | |
| | 1.0 | 7204 | 4.1 | 3 | |
| | 0.5 | 6089 | 3.6 | 19 | |
| | 0.25 | 493 | 1.2 | 2 | |
| | 0.125 | 1118 | 1.5 | 0 | |
| 2. Phenylmercuric acetate£ | 1.0 | -2037 | 0.1 | - | T |
| | 0.5 | 1168 | 1.5 | 22 | |
| | 0.25 | 7986 | 4.4 | 39 | |
| | 0.125 | 770 | 1.3 | .6 | |
| | 0.062 | 2781 | 2.2 | 24 | |
| | 0.031 | 1711 | 1.7 | 34 | |
| 3. $CoCl_2$ £(0,5%) &(1 %) | 20.0 | -548 | 0.8 | 8 | |
| | 10.0 | -980 | 0.6 | 0 | |
| | 5.0 | -640 | 0.7 | 0 | |
| | 2.5 | 799 | 1.3 | 0 | |
| | 1.25 | -893 | 0.6 | 0 | |
| | 0.62 | 95 | 1.0 | 0 | |

Table 11 (Cont.)

| Antigen in culture | Concentration µg/ml | Λcpm | SI | Number of lymphoblasts | |
|---|---|---|---|---|---|
| 4. NiCl$_2$ & | 20.0 | 59562 | 26.7 | >100 | MF |
| | 10.0 | 8826 | 4.8 | 66 | |
| | 5.0 | 6886 | 4.0 | 52 | |
| | 2.5 | 7720 | 4.3 | 16 | |
| | 1.25 | 6762 | 3.9 | 54 | |
| | 0.62 | 8519 | 4.7 | 29 | |
| 5. Gold sodium thiosulfate | 50.0 | 36781 | 16.9 | 62 | |
| | 25.0 | 4637 | 3.0 | 26 | |
| | 12.5 | 8008 | 4.5 | 60 | |
| | 6.25 | 18347 | 8.9 | 86 | |
| | 3.12 | 8904 | 4.8 | 60 | |
| | 1.56 | 4185 | 2.8 | 30 | |
| 6. CrCl$_3$ & | 20.0 | 50662 | 22.9 | >100 | |
| | 10.0 | 75332 | 33.5 | >100 | |
| | 5.0 | 66505 | 29.7 | >100 | |
| | 2.5 | 6684 | 3.9 | 5 | |
| | 1.25 | 4883 | 3.1 | 21 | |
| | 0.62 | 4825 | 3.1 | 6 | |

&=PATCH TEST POSITIVE

£=PATCH TEST NEGATIVE

| | |
|---|---|
| Λcpm | mean cpm of test culture - mean cpm of negative control cultures |
| SI | Stimulation Index |
| PPD | Tuberculin, Purified Protein Derivative |
| * | mean cpm ± standard deviation |
| T | toxic |
| MF | many macrophages |
| ND | not done |

EVALUATION: 1) Strong proliferative response to nickel and chromium salts.
2) Positive response to gold and organic mercury.
3) Negative to cobalt.
4) The results suggest allergy to Cr, Ni, Au and Hg.

Table 12

| LYMPHOCYTE PROLIFERATION TO VARIOUS METALS IN PATIENT VM45 | | | |
|---|---|---|---|
| Antige in culture | Concentration μg/ml | SI | Lymphoblasts* |
| Control | - | 1 | - |
| HgCl$_2$ | 0.125 | 2.1 | - |
| | 0.25 | 0.9 | - |
| | 0.5 | 0.8 | - |
| | 1 | 1.3 | - |
| | 2 | 3.1 | + |
| | 4 | 42 | + |
| Phenyl-mercuric acetate | 0.06 | 1.1 | - |
| | 0.12 | 0.7 | - |
| | 0.25 | 0.8 | - |
| | 0.5 | 3.4 | + |
| | 1.0 | 0.9 | toxic |
| Gold thiosulfate | 1.5 | 16 | + |
| | 3 | 15 | + |
| | 6 | 45 | + |
| | 12 | 97 | + |
| | 25 | 68 | + |
| Titanium chloride | 0.6 | 1.1 | - |
| | 1.2 | 1.3 | - |
| | 2.5 | 1.3 | - |
| | 5 | 2.7 | + |
| | 10 | 0.2 | toxic |
| Cadmium chloride | 3 | 3.6 | + |
| | 6 | 3.8 | + |
| | 12 | 0.1 | toxic - |
| Thimerosal | 0.06 | 0.8 | - |
| | 0.12 | 1.2 | - |
| | 0.25 | 0.9 | - |
| | 0.5 | 0.6 | - |
| | 1 | <0.1 | toxic |
| Nickel chloride | 1.25 | 1 | - |
| | 2.5 | 1.3 | - |
| | 5 | 1.7 | - |
| | 10 | 4.6 | + |
| | 20 | 19 | + |
| * + = positive MELISA, - = negative MELISA | | | |

Table 14A

PATIENT WITH SUSPECTED MERCURY ALLERGY

Patient L 16 - plate incubation

| Antigen | Concentration µg/ml | Λcpm | SI | Number of transformed lymphocytes |
|---|---|---|---|---|
| Control | - | (16864+5407) | 1.0 | 34 |
| | | | | 46 |
| | | | | 18 |
| Positive control PPD | 1.0 | 299779 | 18.8 | >100 |
| HgCl₂ | 4.0 | 22079 | 2.3 | >100 |
| | 2.0 | 32753 | 2.9 | >100 |
| | 1.0 | 32696 | 2.9 | >100 |
| | 0.5 | 30975 | 2.8 | >100 |
| | 0.25 | 59201 | 4.5 | >100 |
| | 0.125 | 50262 | 4.0 | >100 |
| Phenylmercuric acetate | 2.0 | -16531 | <0.1 | - toxic |
| | 1.0 | -11131 | 0.3 | - " |
| | 0.5 | -1180 | 0.9 | 22 |
| | 0.25 | 15151 | 1.9 | >100 |
| | 0.125 | 58994 | 4.5 | >100 |
| | 0.062 | 33939 | 3.0 | >100 |
| Methylmercuric chloride | 2.0 | -16535 | <0.1 | - toxic |
| | 1.0 | -9520 | 0.4 | 20 |
| | 0.5 | -11112 | 0.3 | 24 |
| | 0.25 | 4056 | 1.2 | 90 |
| | 0.125 | 18262 | 2.1 | 90 |
| | 0.062 | 46720 | 3.8 | >100 |

EVALUATION: 1) Positive proliferation to all concentrations of
              HgCl₂ (high and low)
           2) Positive proliferation to phenylmercury and
              methylmercury.
           3) Results show mercury allergy.

Table 14B

PATIENT WITH SUSPECTED MERCURY ALLERGY

Patient S 92 - Plate incubation

| Antigen | Concentration μg/ml | Λcpm | SI | Number of lymphoblasts |
|---|---|---|---|---|
| Control | - | (907±273)* | 1.0 | 1,0,0<br>0,0,0 |
| Positive control<br>PPD | 0.1<br>0.01 | 606793<br>468957 | 670<br>518 | >100<br>>100 |
| HgCl$_2$ | 9<br>3<br>1<br>0.33<br>0.11<br>0.037<br>0.012<br>0.004 | -794<br>5620<br>399<br>226<br>6<br>289<br>-98<br>368 | 0.1<br>6.8<br>1.4<br>1.2<br>1.0<br>1.3<br>0.9<br>1.4 | - toxic<br>4<br>0<br>1<br>1<br>2<br>0<br>0 |
| Phenylmercuric acetate | 0.5<br>0.25<br>0.125<br>0.062<br>0.031<br>0.015<br>0.007 | -128<br>-21<br>100<br>-205<br>-315<br>12<br>-47 | 0.8<br>1.0<br>1.1<br>0.8<br>0.6<br>1.0<br>0.9 | 0<br>0<br>0<br>0<br>0<br>0<br>0 |

EVALUATION:  1) Positive response to high doses of HgCl$_2$ only.
             2) Negative response to phenylmercury.
             3) Results do not indicate mercury allergy.

EP 0 558 566 B1

Table 15

| HEALTHY AMALGAM BEARER ( SK 45 ) - PLATE INCUBATION. | | | | |
|---|---|---|---|---|
| Antigen | Concentration $\mu$g/ml | $\Lambda$cpm | SI | Number of transformed lymphocytes |
| Control | - | (1527±359)* | 1.0 | 0<br>0<br>0 |
| Positive control<br>PPD | 1.0 | 30422 | 20.9 | >100 |
| HgCl$_2$ | 9.0<br>4.5<br>2.25<br>1.12<br>0.56<br>0.28 | -1096<br>2442<br>3642<br>207<br>-289<br>-529 | 0.3<br>2.6<br>3.4<br>1.1<br>0.8<br>0.7 | -<br>10<br>10<br>2<br>2<br>1 |
| Phenylmercuric acetate | 0.5<br>0.25<br>0.125<br>0.062<br>0.031<br>0.015 | -1131<br>56<br>21<br>1574<br>-163<br>-513 | 0.3<br>1.0<br>1.0<br>2.0<br>0.9<br>0.7 | toxic<br>0<br>2<br>0<br>2<br>4 |
| Ethyl mercuric chloride | 0.5<br>0.25<br>0.125<br>0.062 | -1100<br>-667<br>-24<br>1485 | 0.3<br>0.6<br>1.0<br>2.0 | 0<br>0<br>0<br>0 |
| Methyl mercuric chloride | 0.5<br>0.25<br>0.125<br>0.062 | -573<br>-135<br>439<br>422 | 0.6<br>0.9<br>1.3<br>1.3 | 0<br>0<br>0<br>0 |
| EVALUATION:<br>1) Weak proliferation to HgCl$_2$.<br>2) Negative response to organomercurials. | | | | |

## Claims

1. Process for in vitro determination of allergy against an antigen with the help of lymphocytes, so called memory cells, in the blood, by which from the blood isolated lymphocyte fraction is cultivated in the presence of the antigen for the determination of the eventual presence of antigen-specific memory cells among the lymphocytes which is shown by memory cell proliferation, **characterized** in that the lymphocyte fraction being substantially depleted from monocytes and other adherent antigen-presenting cells.

2. Process according to claim 1, **characterized** in that the antigen is a metal, an organic or inorganic metal substance, ceramics, glass ionomers, composite materials or pigments.

3. Process according to claim 2, **characterized** in that the metal is chosen among mercury, platinum, gold, palladium, titan, tin, copper, silver, cobalt, chromium, cadmium, lead, arsenic, aluminium antimony, barium and nickel.

4. Process according to claim 2**, characterized** in that the antigens are one or several organic mercury compounds and/or the low doses (less than 0.6 micrograms per ml when HgCl$_2$ is used) of inorganic

32

mercurials.

5. Process according to claim 2, **characterized** by organical mercury compounds which can be chosen among phenyl, methyl, ethyl, thioethyl or structurally similar organic mercurials.

6. Process according to claim 1, **characterized** by defibrination of the blood or by treatment of blood by anticoagulants which do not disturb presentation of antigenic compounds, followed by gradient centrifugation which yields the cell fraction containing lymphocytes, monocytes and other cells, in which adherent non lymphocytic antigen-presenting cells are removed through the incubation of lymphocyte suspension in a culture container, preferably of plastic, under which a part of the cells adheres to the container walls and non adherent lymphocytes can be removed or in which lymphocyte fraction is mixed with substances toxic for monocytes such as lysosom-tropic agents such as leucine methyl esther or structurally and/or functionally similar substances or in which lymphocote fraction is incubated with substances which prevent the development of monocytes to macrophages such as adenosine or structurally and/or functionally similar substances, and in which the removal of monocytes and other adherent cells is repeated at least twice.

7. Process according to claim 6, **characterized** by removal of monocytes and other adherent cells through the incubation of lymphocyte fraction in culture container, preferably of plastic or other functionally similar material until the cell adherence to the container wall is observed under the invert microscope, preferably 10-15 minutes, after which lymphocytes can be transferred to another wall for an additional adherence. The adherence step is repeated at least twice.

8. Process according to claims 1,5-7, **characterized** by blood defibrination through the shaking of the blood with particles, preferably through the shaking for 5-10 minutes of through the addition of an anticoagulantia which does not interefere with the determination of memory cells.

9. Process according to claims 1,5-8, **characterized** by incubation of lymphocyte-rich fraction in culture vessel, preferably of plastic, in culture medium enriched with serum for 0.5-3 hours, during which monocytes and other adherent cells attach to the vessel wall, following which lymphocytes are transferred to tubes or other cultivation wells in a plate and are cultivated together with the antigen in $CO_2$ incubator containing 5% $CO_2$ in the air, at 100% humidity and 37° for 4-6 days, after which the cells are transferred to fresh tubes or wells and incubated with radioactive precursor which can be incorporated into DNA, RNA or protein, preferably $^3$H-methyl thymidine at 37°C during the 3-5 hours in the same incubator and in which parallelly another sample is taken from each lymphocyte culture for morphological evaluation of stimulated lymphoblasts after cytocentrifugation, fixation with methanol and staining with May-Grundwald and Giemsa or other staining generally used for similar purposes or with the help of monoclonal antibodies against stimulated lymphocytes by flow cyto fluorometry.

10. The use of the process according to claims 1,5-9 for analysis of the mechanisms underlying or contributing to the diseases which may have metal allergy ethiology.

**Patentansprüche**

1. Verfahren zur In-Vitro-Bestimmung einer Allergie auf ein Antigen mit Hilfe von Lymphozyten, sogenannten Memoryzellen, im Blut, bei dem eine vom Blut isolierte Lymphozytenfraktion kultiviert wird in Anwesenheit des Antigens zur Bestimmung der eventuellen Anwesenheit von antigen-spezifischen Memoryzellen zwischen den Lymphozyten, was durch Memoryzellensprossung gezeigt wird, dadurch gekennzeichnet, daß die Lymphozytenfraktion im wesentlichen von Monozyten und anderen adhärenten antigenzeigenden Zellen befreit ist.

2. Verfahren nach Anspruch 1, dadurch gekennzeichnet, daß das Antigen ein Metall ist, eine organische oder anorganische Metallsubstanz, Keramik, Glasionemere, zusammengesetzte Materialien oder Pigmente.

3. Verfahren nach Anspruch 2, dadurch gekennzeichnet, daß das Metall aus Quecksilber, Platin, Gold, Palladium, Titan, Zinn, Kupfer, Silber, Kobalt, Chrom, Cadmium, Blei, Arsen, Aluminumantimon, Barium und Nickel gewählt wird.

4. Verfahren nach Anspruch 2, dadurch gekennzeichnet, daß die Antigene einzelne oder mehrere organische Quecksilberkomponenten und/oder eine geringe Dosis (weniger als 0,6 Mikrogramm pro ml wenn $HgCl_2$ verwendet wird) von anorganischen Quecksilberkomponenten sind.

5. Verfahren nach Anspruch 2, charakterisiert durch organische Quecksilberkomponenten, die aus Phenyl, Methyl, Ethyl, Thioethyl oder struktural ähnlichen organischen Quecksilberkomponenten gewählt werden können.

6. Verfahren nach Anspruch 1, gekennzeichnet durch die Defibrination von Blut oder durch die Behandlung von Blut mit Anticoagulantien, die die Anwesenheit von Antigenkomponenten nicht stören, gefolgt durch eine Gradientenzentrifugation, die die Zellfraktion ergibt, die Lymphozyten, Monozyten und andere Zellen enthält, in der adhärente nicht-lymphozytische antigen-zeigende Zellen entfernt sind durch die Inkubation der Lymphozytensuspension in einem Kulturgefäß, vorzugsweise aus Kunststoff, bei dem ein Teil der Zellen an der Gefäßwand haftet, und nicht anhaftende Lymphozyten entfernt werden können, oder in dem die Lymphozytenfraktion mit für Monozyten toxischen Substanzen gemischt werden, wie z.B. Lysosom-tropic Agenzien wie zum Beispiel Leucin-methylester oder struktural und/oder funktional ähnlichen Substanzen oder dem die Lymphozytenfraktion mit Substanzen inkubiert wird, die die Entwicklung von Monozyten zu Macrophagen verhindert wie zum Beispiel Adenosine, oder struktural und/oder funktional ähnlichen Substanzen, und in dem die Entfernung von Monozyten und anderen adhärenten Zellen wenigstens zweimal wiederholt wird.

7. Verfahren nach Anspruch 6, gekennzeichnet durch die Entfernung von Monozyten und anderen adhärenten Zellen durch Inkubation der Lymphozytenfraktion in einem Kulturgefäß, vorzugsweise aus Kunststoff oder einem anderen funktional ähnlichen Material bis die Zelladhärenz an der Gefäßwand unter dem Invert-Mikroskop beobachtet wird, vorzugsweise 10 bis 15 Minuten, nachdem die Lymphozyten zu einer anderen Wand transferriert werden können für eine zusätzliche Adhärenz. Der Adhärenzschritt wird wenigstens zweimal wiederholt.

8. Verfahren nach den Ansprüchen 1, 5-7, charakterisiert durch die Blutdefibrination durch das Schütteln des Bluts mit Partikeln, vorzugsweise durch das Schütteln 5 bis 10 Minuten lang durch die Zugabe von Anticoagulantien, die die Bestimmung der Memoryzellen nicht stören.

9. Verfahren nach den Ansprüchen 1, 5-8, gekennzeichnet durch die Inkubation der lymphozyten-reichen Fraktion in einem Kulturgefäß, vorzugsweise aus Kunststoff, in einem Kulturmedium, das 0,5 bis 3 Stunden mit Serum angereichert wird, währenddessen Monozyten und andere adhärente Zellen an der Gefäßwand anhaften, wobei nachfolgend die Lymphozyten zu Röhren oder anderen Kultivationsbehältern in einer Platte überführt werden und zusammen mit dem Antigen in einem $CO_2$ Inkubator, der 5 % $CO_2$ in der Luft enthält, bei 100 % Feuchtigkeit und 37° vier bis sechs Tage lang kultiviert werden, und die Zellen danach in frische Röhre oder Behälter überführt werden, und mit radioaktiven Precursoren inkubiert werden, die in DNA, RNA oder Protein incorporiert werden können, vorzugsweise $^3$H-Methylthymidin bei 37° C während drei bis fünf Stunden in demselben Inkubator und in dem parallel eine andere Probe genommen wird von jeder Lymphozytenkultur zur morphologischen Bestimmung der stimulierten Lymphoplasten nach Cytozentrifugation, Fixierung mit Methanol und Einfärbung mit May-Grundwald und Giemsa oder anderen Färbungen, die im allgemeinen für ähnliche Zwecke benutzt werden, oder mit Hilfe von monoclonalen Antikörpern gegen stimulierte Lymphozyten durch Flowcytofluormetrie.

10. Verwendung des Verfahrens gemäß den Ansprüchen 1, 5-9 zur Analyse des Mechanismus, der den Krankheiten zugrundeliegt oder dazu beiträgt, die eine Metallallergie-Ätiologie haben können.

**Revendications**

1. Procédé de détermination in vitro d'une allergie à un antigène à l'aide de lymphocytes, dénommés cellules à mémoire, du sang, qui consiste à cultiver une fraction de lymphocytes isolés du sang en la présence de l'antigène pour la détermination de la présence éventuelle de cellules à mémoire spécifiques de l'antigène parmi les lymphocytes, ce qui est indiqué par une prolifération de cellules à mémoire, caractérisé en ce qu'il consiste à sensiblement épuiser la fraction de lymphocytes de monocytes et d'autres cellules adhérentes présentant un antigène.

2. Procédé suivant la revendication 1, caractérise en ce que l'antigène est un métal, une substance métallique organique ou minérale, des céramiques, des ionomères vitreux, des matières composites ou des pigments.

3. Procédé suivant la revendication 2, caractérisé en ce que le métal est choisi parmi le mercure, le platine, l'or, le palladium, le titane, l'étain, le cuivre, l'argent, le cobalt, le chrome, le cadmium, le plomb, l'arsenic, l'aluminium, l'antimoine, le baryum et le nickel.

4. Procédé suivant la revendication 2, caractérisé en ce que les antigènes sont un ou plusieurs composés organo-mercuriens et/ou de faibles doses (inférieures à 0.6 microgrammes par ml quand on utilise $HgCl_2$) de composés minéraux du mercure.

5. Procédé suivant la revendication 2, caractérisé par des composés organomercuriens qui peuvent être choisis parmi des phénylmercuriens, méthylmercuriens, éthylmercuriens, thioéthylmercuriens ou des composés organomercuriens de structure similaire.

6. Procédé suivant la revendication 1, caractérisé par une défibrination du sang ou par un traitement du sang par des anticoagulants qui ne gênent pas la présence de composés antigéniques, suivi d'une centrifugation avec gradient qui donne la fraction cellulaire contenant des lymphocytes, des monocytes et d'autres cellules, dans lequel des cellules adhérentes non lymphocytiques comportant des antigènes sont éliminées par l'incubation d'une suspension de lymphocytes dans un récipient de culture, de préférence en matière plastique, une partie des cellules adhérant aux parois du récipient et les lymphocytes non adhérents pouvant être éliminés ou dans lequel une fraction de lymphocytes est mélangée à des substances toxiques pour des monocytes telles que des agents lysosome-tropiques comme l'ester de leucine méthyle ou des substances similaires structurellement et/ou fonctionnellement ou dans lequel une fraction de lymphocytes est incubée avec des substances qui empêchent la transformation de monocytes en macrophages telles que l'adénosine ou des substances similaires structurellement et/ou fonctionnellement, et dans lequel l'élimination de monocytes et d'autres substances adhérentes est répétée au moins deux fois.

7. Procédé suivant la revendication 6, caractérisé par une élimination des monocytes et d'autres cellules adhérentes par l'incubation d'une fraction de lymphocytes dans un récipient de culture, de préférence en matière plastique ou en une autre matière similaire fonctionnellement jusqu'à ce qu'on observe l'adhérence des cellules à la paroi du récipient au microscope inversé, de préférence pendant 10 à 15 minutes, après quoi des lymphocytes peuvent être transférés à une autre paroi en vue de les faire encore adhérer. Le stade d'adhérence est répété au moins deux fois.

8. Procédé suivant les revendications 1 ou 5 à 7, caractérisé par une défibrination du sang en agitant le sang avec des particules, de préférence en agitant pendant 5 à 10 minutes ou par l'addition d'anticoagulants qui n'interfèrent par avec la détermination des cellules à mémoire.

9. Procédé suivant les revendications 1 ou 5 à 8, caractérisé par une incubation d'une fraction riche en lymphocytes dans un récipient de culture, de préférence en matière plastique, dans un milieu de culture enrichi en sérum pendant 0.5 à 3 heures, pendant laquelle des monocytes et d'autres cellules adhérentes se fixent à la paroi du récipient, puis en faisant suivre d'un transfert des lymphocytes à des tubes ou autres puits de culture dans une plaque et d'une culture de ceux-ci avec l'antigène dans un incubateur à $CO_2$ contenant 5% de $CO_2$ dans l'air à 100% d'humidité et à 37°C pendant 4 à 6 jours, après quoi les cellules sont transférées dans des tubes ou dans des puits nouveaux et incubées par un précurseur radioactif qui peut être incorporé à de l'ADN, à de l'ARN ou à une protéine, de préférence de la $^3$H-méthyl thymidine à 37°C pendant les 3 à 5 heures dans le même incubateur et dans lequel parallèlement on prélève un autre échantillon de chaque culture de lymphocyte pour une évaluation morphologique de lymphoblastes stimulés après cytocentrifugation, fixation au méthanol et coloration par May-Grundwald et Giemsa ou d'autres agents de coloration utilisés en général à des fins similaires ou à l'aide d'anticorps monoclonaux contre des lymphocytes stimulés par cytofluorométrie d'écoulement.

10. L'utilisation du procédé suivant les revendications 1 ou 5 à 9 pour l'analyse du mécanisme sous-jacent ou contribuant aux maladies dont l'étiologie peut être due à une allergie à un métal.

LYMPHOCYTE PROLIFERATION TO INORGANIC AND ORGANIC MERCURIALS IN PATIENTS WITH ORAL LICHEN

CLOSED SYMBOLS: POSITIVE MELISA
OPEN SYMBOLS: NEGATIVE MELISA

S PATIENTS

FIG.1

EP 0 558 566 B1

## SUBJECTS TOLERATING AMALGAM

FIG. 2

CLOSED SYMBOLS: POSITIVE MELISA
OPEN SYMBOLS: NEGATIVE MELISA

# LYMPHOCYTE PROLIFERATION TO INORGANIC AND ORGANIC MERCURIALS IN AMALGAM—FREE DONORS

STIMULATION INDEX

Legend:
- $HgCl_2 \leq 0.5\mu g$
- $HgCl_2 \geq 1\mu g$
- PHENYL- Hg

POSITIVE INCORPORATION

CLOSED SYMBOLS: POSITIVE MELISA
OPEN SYMBOLS: NEGATIVE MELISA

TL PATIENTS

FIG. 3

EP 0 558 566 B1

LYMPHOCYTE PROLIFERATION TO MERCURY AND OTHER METALS IN PATIENT WITH ORAL LICHEN (S31)

FIG.4

$\mu$G/ML

N=NICKEL   C=COBALT

TL31 — DONOR TOLERATING AMALGAM

FIG. 5

EP 0 558 566 B1

TL60 — AMALGAM FREE DONOR

FIG.6

LYMPHOCYTE PROLIFERATION TO VARIOUS METALS IN PATIENTS
WITH ORAL SYMPTOMS RELATED TO GOLD FILLINGS (S85) **

* broken line indicates borderline of positive
  response.
**Patients symptoms disappeared after removal
  of gold fillings(clinical relevance to gold).

FIG.7